# EUROPEAN PATENT APPLICATION

(11) **EP 3 338 785 A1**
(43) Date of publication of application: **27.06.2018**
(21) Application number: 16205996.8
(22) Date of filing: 21.12.2016
(51) Int. Cl.: A61K 35/747, G01N 33/48, C12N 1/20, A61P 31/04

(54) **HUMAN LACTOBACILLI STRAINS**

(71) Applicant: Agriculture and Food Development Authority (TEAGASC), Co. Carlow (IE); University College Cork - National University of Ireland, Cork, Cork City (IE)
(72) Inventor: HILL, Colin, Cork, (IE); ROSS, Paul, County Cork, (IE); REA, Mary Clare, County Cork, (IE); ALEMAYEHU, Debebe, County Cork, (IE); MURPHY, Eileen Frances, Cork (IE)
(74) Representative: O'Brien, John Augustine

(57) **Abstract**

*Clostridium difficile* is one of the most common causes of health-care acquired diarrhoea, resulting in a spectrum of disease from mild diarrhoea to life-threatening illness. Particular human *Lactobacillus* strains, including *Lactobacillus gasseri* APC678, are described as useful therapeutic agents to target *C. difficile* colonisation or infection.

## Description

### Introduction

*Clostridium difficile* is a Gram positive, cytotoxin-producing anaerobic intestinal pathogen with an asymptomatic carriage rate of up to 30 % of people in long-term care facilities (Ziakas *et al.,* 2015). However, when the intestinal microbiota is altered following broad spectrum antibiotic therapy, *C*. *difficile* may flourish and cause illness varying from mild diarrhoea (usually self-limiting) to pseudomembraneous colitis, fulminant colitis, toxic megacolon and even death (Kachrimanidou and Malisiovas, 2011). The incidences of *C. difficile* infection (CDI) have rapidly increased since the 1990s, and the mortality rate has also grown markedly (Wiegand *et al.,* 2012). Recent studies indicate that the economic burden of *C. difficile* is mounting as a result of increased number of cases of infection in hospitalised patients. Latest estimates show that the economic health-care costs of CDI are over $ 4.8 billion per annum in the U.S. and over € 3 billion per annum in Europe (DePestel and Aronoff, 2013b).

The European Society of Clinical Microbiology and Infection (ESCMID) guidelines for treatment of CDI include antibiotics, toxin-binding resins and polymers, immunotherapy, probiotics and faecal or bacterial intestinal transplantation (Debast *et al.,* 2014). Antibiotic treatment is typically advised, including the use of metronidazole, vancomycin and fidaxomicin (Debast *et al.,* 2014). However, as standard therapies for CDI frequently have limited efficacy, the search for alternative therapies such as live therapeutics and bacteriocins that may help to reduce incidences and recurring infections are gaining credence (Evans and Johnson, 2015; Goldstein *et al.,* 2015; Rea *et al.,* 2013). One of the strategies used to modulate the gut microbiota is the dietary administration of live microorganisms, (Hill et al., 2014). A meta-analysis of the literature, from 1985 to 2013, relating to the ability of probiotics to prevent paediatric antibiotic-associated diarrhoea and CDI found that the use of probiotics significantly prevented CDI and antibiotic-associated diarrhoea in children, but that the effect of probiotics was strain dependant (McFarland and Goh, 2013). The mode of action of live microbes is multifaceted and includes an improvement of epithelial barrier function, immune-modulation, secretion of antimicrobial substances (e.g. bacteriocins and H₂O₂), and bioactive metabolites (e.g. CLA), inhibition of the expression of virulence factors, playing a role in competitive exclusion possibly through colonization resistance or through the production of neurotransmitters such as Gamma-aminobutyric acid (GABA) which may impact on brain function (Dinan *et al.,* 2015; Dobson *et al.,* 2012; Fernandez *et al.,* 2015; Nebot-Vivinus *et al.,* 2014; O'Shea *et al.,* 2012; Sultana *et al.,* 2013). However, the effect of pure cultures of bacterial strains administered as live therapeutics has been shown in many instances to be strain rather than species dependant indicating that careful strain selection is required (Wall et al 2012).

The effect of bacteria frequently associated with the human gut, such as *C. difficle* is being increasingly understood within the whole context of the human gut microbiome. The human gut microbiota consists of greater than 1000 bacterial species, with every individual hosting at least 160 different species (Eckburg *et al.,* 2005; Qin *et al.,* 2010; Tap *et al.,* 2009; Walker *et al.,* 2011). The gut microbiota of a healthy adult is, in general, stable with *Firmicutes* and *Bacteroidetes* typically accounting for over 90% of bacteria with smaller fractions of *Actinobacteria* and *Proteobacteria* (Eckburg *et al.,* 2005; Claesson *et al.,* 2011; Ley *et al.,* 2006; Human Microbiome Project Consortium, 2012). However, considerable inter-individual variability exists at species and strain level (Lozupone *et al.,* 2012). Although it is not yet fully clear what constitutes a 'healthy' gut microbiota diversity and composition are key factors (Backhed *et al.,* 2012; Claesson *et al.,* 2012; Jeffery *et al.,* 2016; Le Chatelier *et al.,* 2013) and a gut microbiota with low diversity may have negative consequences for health (Thomas *et al.,* 2014). This low microbial diversity can arise in several human life stages or due particular therapeutic interventions.

A recent study found the indigenous Yanomami people in the Amazon, who had no contact with Western civilization, to have the richest and most diverse microbiota ever recorded in humans (Clemente *et al.,* 2015). Additionally, they had high levels of bacteria such as *Prevotella, Helicobacter, Oxalobacter* and *Spirochaeta,* which are hardly present or present in very low levels in people living in the United States (US). Furthermore, a similar study which compared the faecal microbiota of adults from two non-industrialized regions of Papua New Guinea with that of individuals from the US found the gut microbiota of the former had greater bacterial diversity, lower inter-individual variation, vastly different abundance profiles and bacterial lineages undetectable in the US individuals (Martínez *et al.,* 2015). These studies suggest that Westernization significantly affects human microbiota diversity and that modern lifestyle impacts the biodiversity of our microbiota and that specific positive functions, such as proper immune priming in infancy are probably lost. This may also explain the growth in recent decades of autoimmune diseases and metabolism-related disorders in Western civilization which are almost non-existent in non-industrialized countries.

Noncommunicable diseases (NCDs), also known as chronic diseases, are diseases that are not caused by infectious agents and are not passed from person to person. They are generally of long duration and slow progression (WHO, 2016) and include diseases such as autoimmune disease, cardiovascular disease, stroke, cancer, osteoporosis, depression, anxiety, autism, Alzheimer's disease, chronic kidney disease, diabetes and obesity. NCDs are the leading cause of death globally (WHO, 2016).

Reduced microbial diversity is centrally implicated in many NCDs. Studies have demonstrated associations between reduced microbial diversity and eczema (Bisgaard et al., 2011; Abrahamsson et al., 2012; Ismail et al., 2012; van Nimwegen et al., 2011), asthma (Ege et al., 2011; Abrahamsson et al., 2014) autoimmune disease (Kostic et al., 2015; Knip & Siljander, 2016), cardiovascular disease (Kelly et al., 2016) and obesity and type 2 diabetes (Le Chatelier et al., 2013; Remely et al., 2014; Turnbaugh et al., 2009). Reduced diversity has been observed in patients with Crohn's disease (Sokol et al., 2008), type 1 diabetes, coeliac disease, allergy, autism and cystic fibrosis (Spor et al., 2011), all non-communicable diseases. Reduced diversity has also been associated with an increased risk of adiposity, insulin resistance, high blood lipid levels and inflammation (Cotillard *et al.,* 2013; Le Chatelier *et al.,* 2013) - features that can lead to Non-alcoholic liver disease (NAFLD) and its more serious follow-on Non-alcoholic steatohepatitis (NASH) as well as contributing to many of the disease highlighted above.

Obesity as a growing health issue and again it has been highlighted that the diversity of the gut microbiota has relevance. Gut microbiota from obese mice had a lower bacterial diversity than that from lean mice (Turnbaugh *et al.,* 2008). Similarly, 16S rRNA gene surveys revealed a reduced diversity of gut microbiota in human obese twins compared to their lean twin counterparts (Turnbaugh *et al.,* 2009).

Furthermore, gut microbiota diversity may be a contributing factor to disorders of brain function, such as depression, anxiety and autism, through bidirectional signalling via the gut-brain axis (Collins *et al.,* 2012; Cryan and Dinan, 2012; Mayer *et al.,* 2014; Mayer *et al.,* 2015; Stilling *et al.,* 2014).

Finally, compositional diversity also decreases as we age (Claesson *et al.,* 2011), which has been associated with a less diverse diet and correlates with poor health, increased frailty and markers of inflammation (Claesson *et al.,* 2012).

The impact of the microbiota also extends to infectious disease. Individuals with a less diverse gut microbiota are more susceptible to antibiotic-associated dysbiosis (O'Sullivan *et al.,* 2013). A reduction in diversity was observed in patients with *Clostridium difficile (C. difficile)* associated diarrhoea while asymptomatic subjects from whom *C. difficile* was isolated showed no significant difference in diversity compared to the control group. *C. difficile* infection is normally the result of perturbation of the gut microbiota as a result of broad-spectrum antibiotic treatment which results in a decrease in diversity of the gut microbiota (Rea *et al.,* 2012a). Introducing a probiotic strain in a disease state could increase diversity, thus reducing the ability of *C. difficile* to survive and multiply due to competition for nutrients.

Older adults (>65 years) have an increased risk of *C. difficile* infection (Goorhuis *et al.,* 2008; Vardakas *et al.,* 2012). Additionally, the risk of hospitalization associated with a *C. difficile* infection increases with age (Lucado *et al.,* 2006). Although *C. difficile* has historically been considered a nosocomial pathogen associated with antibiotic exposure *C. difficile* infections have emerged in populations previously considered low risk, such as healthy peripartum women, children, antibiotic-naive patients, and those with minimal or no recent healthcare exposure (DePestel and Aronoff, 2013a; Kim *et al.,* 2008; Wilcox *et al.,* 2008). This expansion of disease prevalence and virulence highlights the unmet need for safe, effective treatments for both acute infections and to maintain the health status of at-risk populations.

Recently faecal microbiota transplantation (FMT) has also been used with some success for the treatment of refractory CDI and the interest in this area has risen as evidenced by the large increase in publications relating to FMT over the last 3 years (Bojanova and Bordenstein 2016). To date the mechanism of action of FMT to break the cycle of recurrent CDI remains poorly understood. However, intra colonic bile acid has been suggested to play a role in both spore germination and inhibition of vegetative cells of *C. difficile* (Weingarden et al 2016). *Clostridium scindens,* a member of the intestinal microbiota capable of dehyrdoxylating bile acid, was shown to be associated with resistance to *C. difficile* and enhanced resistance to infection in a murine model of CDI (Buffie et al 2015).

Although, in the short-term, serious adverse effects directly attributable to FMT in patients with normal immune function are uncommon the long-term safety of FMT is unknown (Rao and Safdar, 2016). The interactions between the gut microbiome and the host are complex system and associations with disease processes have been demonstrated. FMT may therefore have unintended consequences in a patient after successful FMT due to alteration of the gut microbiota. FMT in experimental animals has shown that immunologic, behavioural and metabolic phenotypes can be transferred from donor to recipient ((Collins *et al.,* 2013; Di Luccia *et al.,* 2015; Pamer, 2014). This raises questions about the selection of FMT donors. Since FMT in humans has been shown to transfer an improved metabolic phenotype from lean donors to individuals with metabolic syndrome (Vrieze *et al.,* 2012), the reverse could also be true. This implies that donor selection for FMT should not be based solely on exclusion of transmissible infections (Shanahan, 2015).

In summary there is an ongoing need to provide therapies for prophylaxis and/or treatment of *C. difficile* infection, post-antibiotic infection and non-communicable diseases that are caused by or exacerbated by perturbations and reduced diversity in the human gut microbiome

### Statements of Invention

According to the invention there is provided a strain selected from one or more of:-
*Lactobacillus gasseri* strain APC678 having NCIMB accession number 42658;
*Lactobacillus rhamnosus* DPC6111 having NCIMB accession number 42661;
*Lactobacillus gasseri* strain DPC6112 having NCIMB accession number 42659; and
*Lactobacillus paracasei* strain APC1483 having NCIMB accession number 42660 for use in the prophylaxis and/or treatment of a *Clostridium difficile* colonisation or infection.

The invention also provides a formulation comprising one or more strains selected from:
*Lactobacillus gasseri* strain APC678 having NCIMB accession number 42658;
*Lactobacillus rhamnosus* DPC6111 having NCIMB accession number 42661;
*Lactobacillus gasseri* strain DPC6112 having NCIMB accession number 42659; and
*Lactobacillus paracasei* strain APC1483 having NCIMB accession number 42660.

The formulation may comprise an ingestible carrier.

The ingestible may be a pharmaceutically acceptable carrier.

In some cases the ingestible carrier is a food product.

The food product may be selected from the group comprising acidified milk, yoghurt, frozen yoghurt, milk powder, milk concentrate, cheese spread, dressing and beverage.

In one case the formulation is in the form of a fermented food product.

In one embodiment the formulation is in the form of a fermented milk product.

In some embodiments the carrier does not occur in nature.

In some cases the formulation is in the form of a capsule, a tablet, a pellet, or a powder.

In some embodiments the strain is present in the formulation at more than 10⁶ cfu per gram of ingestible carrier.

The invention also provides *Lactobacillus gasseri* strain APC678 having NCIMB accession number 42658 or mutants or variants thereof.

The invention also provides *Lactobacillus rhamnosus* DPC6111 having NCIMB accession number 42661 or mutants or variants thereof.

The invention also provides *Lactobacillus gasseri* strain DPC6112 having NCIMB accession number 42659 or mutants or variants thereof.

The invention also provides *Lactobacillus paracasei* strain APC1483 having NCIMB accession number 42660 or mutants or variants thereof.

In some embodiments the mutant is a genetically modified mutant.

In some embodiments the variant is a naturally occurring variant.

The strain(s) may be probiotic.

The strain may be in the form of a biologically pure culture.

The invention also provides an isolated strain of *Lactobacillus gasseri* APC678 (NCIMB 42658).

The invention also provides an isolated strain of *Lactobacillus rhamnosus* DPC6111 (NCIMB 42661).

The invention also provides an isolated strain of *Lactobacillus gasseri* DPC6112 (NCIMB 42659).

The invention also provides an isolated strain of *Lactobacillus paracasei* APC1483 (NCIMB 42660).

The strain may be in the form of viable cells.

The strain may be in the form of non-viable cells.

In some cases the strain is isolated from human faeces.

In some embodiments the strain is in the form of a bacterial broth.

In some cases the strain is in the form of a freeze-dried powder.

The invention also provides a formulation comprising at least one isolated strain of the invention.

The formulation may comprise an ingestible carrier.

The ingestible may be a pharmaceutically acceptable carrier.

In some cases the ingestible carrier is a food product.

The food product may be selected from the group comprising acidified milk, yoghurt, frozen yoghurt, milk powder, milk concentrate, cheese spread, dressing and beverage.

In one case the formulation is in the form of a fermented food product.

In one embodiment the formulation is in the form of a fermented milk product.

In some embodiments the carrier does not occur in nature.

In some cases the formulation is in the form of a capsule, a tablet, a pellet, or a powder.

In some embodiments the strain is present in the formulation at more than 10⁶ cfu per gram of ingestible carrier.

The invention also provides a vaccine comprising one or more of the strains of the invention

The strain or a formulation may be for use as a probiotic.

The strain or a formulation may be for the prophylaxis and/or treatment of a *Clostridium difficile* colonisation or infection.

The invention also provides a method for the prophylaxis and/or treatment of a *Clostridium difficile* colonisation or infection comprising administering a strain or a formulation of the invention.

The invention further provides a method for screening a bacterial strain for activity against *C. difficile* comprising:
co-culturing a strain of interest with *C. difficile* in a co-culture medium comprising sugars (such as glucose) in a concentration of from 0.01 g/l to 2 g/l, the co-culture medium having a pH of from 6.7 to 6.9.

The invention also provides a method for screening a bacterial strain for activity against *C. difficile* comprising:
co-culturing a strain of interest with *C. difficile* in a co-culture medium comprising sugars (such as glucose) in a concentration of from 0.01 g/l to 01.0 g/l, the co-culture medium having a pH of 6.8

In some cases the co-culture medium comprises sugars (such as glucose) in a concentration of from 0.05 g/l to 0.5 g/l.

The co-culture medium in some cases comprises sugars (such as glucose) in a concentration of from 0.09 g/l to 0.11 g/l.

The strain of interest may, for example, be a *Lactobacillus.*

The invention also provides a co-culture medium for use in screening a bacterial strain for activity against *C. difficile* wherein the medium comprises sugars (such as glucose) in a concentration of from 0.01 g/l to 2.0 g/l and wherein the co-culture medium has a pH of from 6.7 to 6.9.

Also provided is a co-culture medium for use in screening a bacterial strain for activity against *C. difficile* wherein the medium comprises sugars (such as glucose) in a concentration of from 0.01 g/l to 1.0 g/l and wherein the co-culture medium has a pH of 6.8.

In some cases the co-culture medium comprises sugars (such as glucose) in a concentration of from 0.05 g/l to 0.5 g/l.

The co-culture medium in some cases comprises sugars (such as glucose) in a concentration of from 0.09 g/l to 0.11g/l.

The invention also provides a method of increasing the bacterial biodiversity in the gastrointestinal tract comprising the step of administering to a subject a strain or a formulation of the invention.

In some embodiments the strain or formulation is administered in association with antibiotic treatment.

In some embodiments the subject has a compromised immune system.

The subject may be an elderly patient who may be more than 65 years of age.

The strain or formulation may be for use in the prophylaxis or treatment of low bacterial biodiversity of the gastrointestinal tract.

The isolated strains may be in the form of viable cells.

The isolated strains may be in the form of non-viable cells.

Formulations comprising one or more of the strains may also comprise an ingestible carrier. The ingestible carrier may be a pharmaceutically acceptable carrier such as a capsule, tablet or powder. The ingestible carrier may be a food product such as acidified milk, yoghurt, frozen yoghurt, milk powder, milk concentrate, cheese spreads, dressings or beverages.

The strains of the invention may be administered to animals (including humans) in an orally ingestible form in a conventional preparation such as capsules, microcapsules, tablets, granules, powder, troches, pills, suppositories, suspensions and syrups. Suitable formulations may be prepared by methods commonly employed using conventional organic and inorganic additives. The amount of active ingredient in the medical composition may be at a level that will exercise the desired therapeutic effect.

A formulation comprising one or more of the strains may also include a bacterial component, a drug entity or a biological compound.

In addition a vaccine comprising at least one strain of the invention may be prepared using any suitable known method and may include a pharmaceutically acceptable carrier or adjuvant.

The invention also includes mutants and variants of the deposited strains. Throughout the specification the terms mutant, variant and genetically modified mutant include a strain whose genetic and/or phenotypic properties are altered compared to the parent strain. Naturally occurring variant includes the spontaneous alterations of targeted properties selectively isolated. Deliberate alteration of parent strain properties is accomplished by conventional (*in vitro*) genetic manipulation technologies, such as gene disruption, conjugative transfer, etc. Genetic modification includes introduction of exogenous and/or endogenous DNA sequences into the genome of a strain, for example by insertion into the genome of the bacterial strain by vectors, including plasmid DNA, or bacteriophages.

Natural or induced mutations include at least single base alterations such as deletion, insertion, transversion or other DNA modifications which may result in alteration of the amino acid sequence encoded by the DNA sequence.

The terms mutant, variant and genetically modified mutant also include a strain that has undergone genetic alterations that accumulate in a genome at a rate which is consistent in nature for all micro-organisms and/or genetic alterations which occur through spontaneous mutation and/or acquisition of genes and/or loss of genes which is not achieved by deliberate (*in vitro*) manipulation of the genome but is achieved through the natural selection of variants and/or mutants that provide a selective advantage to support the survival of the bacterium when exposed to environmental pressures such as antibiotics. A mutant can be created by the deliberate (*in vitro*) insertion of specific genes into the genome which do not fundamentally alter the biochemical functionality of the organism but whose products can be used for identification or selection of the bacterium, for example antibiotic resistance.

A person skilled in the art would appreciate that mutant or variant strains can be identified by DNA sequence homology analysis with the parent strain. Strains having a close sequence identity with the parent strain without demonstrable phenotypic or measurable functional differences are considered to be mutant or variant strains. A strain with a sequence identity (homology) of 99.5% or more with the parent DNA sequence may be considered to be a mutant or variant. Sequence homology may be determined using on-line homology algorithm "BLAST" program, publicly available at http://www.ncbi.nlm.nih,gov/BLAST/.

Mutants of the parent strain also include derived strains having at least 95.5% sequence homology to the 16S rRNA polynucleotide sequence of the parent strain. These mutants may further comprise DNA mutations in other DNA sequences in the bacterial genome.

### Brief Description of the Drawings

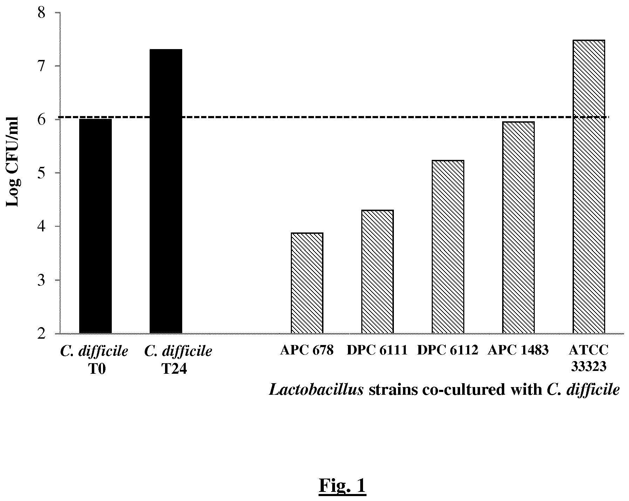

The invention will be more clearly understood from the following description thereof, given by way of example only, in which:-
Fig. 1 is Effect of lactobacilli on the survival of *Clostridium difficile* in co-culture. Black bars (■) show the cell numbers of *C. difficile* at T0 and T 24 in the absence of *Lactobacillus* strains. Bars with dashed lines ( 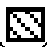 ) show the cell numbers of *C. difficile* following 24 h co-culture with: *L. gasseri* APC 678, *L. rhamnosus* DPC 6111, *L. gasseri* DPC 6112, *L. paracasei* APC 1483 or *L. gasseri* ATCC 33323. The horizontal dashed line (---) indicates the starting inoculum of C. *difficile* at 0 h; and

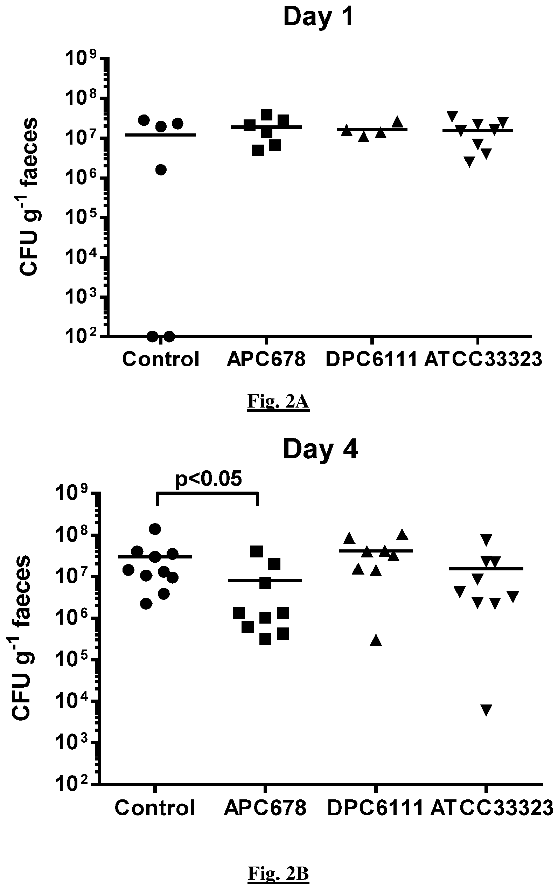

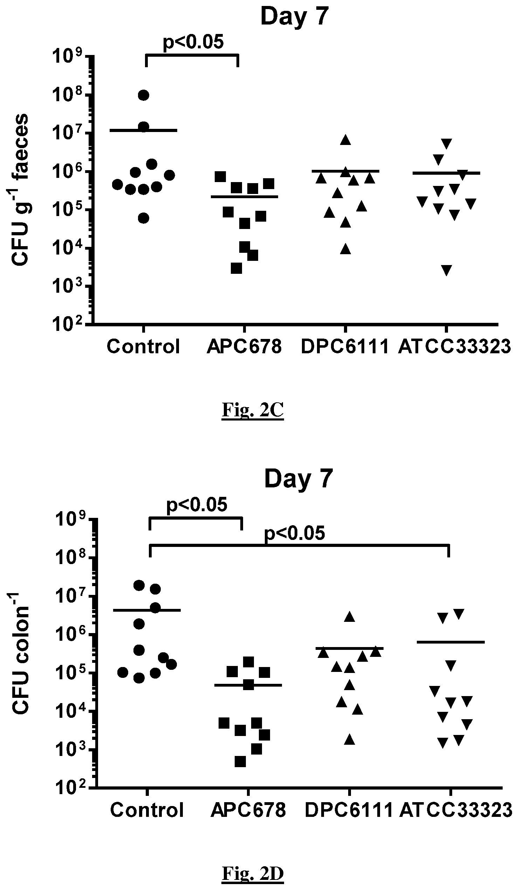

Fig. 2 is *Clostridium difficile* detected during faecal shedding. *C. difficile* detected in mouse faeces (CFU g⁻¹ faeces) following (a) 24 h, (b) 4 days, (c) 7 days administration of lactobacillus strains and (d) *C. difficile* levels in mouse colon (CFU colon⁻¹) following 7 days injestion of the lactobacillus strains (or control). Control: 10 % RSM only; *Lactobacillus gasseri* APC 678; *Lactobacillus rhamnosus* DPC 6111 and *Lactobacillus gasseri* ATCC 33323;

### Detailed Description

A deposit of *Lactobacillus gasseri* strain APC678 was made at the National Collections of Industrial and Marine Bacteria Limited (NCIMB) Ferguson Building, Craibstone Estate, Bucksburn, Aberdeen, AB21 9YA, Scotland, UK on September 20, 2016 and accorded the accession number NCIMB42658.

A deposit of *Lactobacillus rhamnosus* strain DPC6111 was made at the National Collections of Industrial and Marine Bacteria Limited (NCIMB) Ferguson Building, Craibstone Estate, Bucksburn, Aberdeen, AB21 9YA, Scotland, UK on September 20, 2016 and accorded the accession number NCIMB 42661.

A deposit of *Lactobacillus gasseri* strain DPC6112 was made at the National Collections of Industrial and Marine Bacteria Limited (NCIMB) Ferguson Building, Craibstone Estate, Bucksburn, Aberdeen, AB21 9YA, Scotland, UK on September 20, 2016 and accorded the accession number NCIMB 42659.

A deposit of *Lactobacillus paracasei* strain APC1483 was made at the National Collections of Industrial and Marine Bacteria Limited (NCIMB) Ferguson Building, Craibstone Estate, Bucksburn, Aberdeen, AB21 9YA, Scotland, UK on September 20, 2016 and accorded the accession number NCIMB 42660.

### Screening method for anti-C. difficile probiotics.

Lactobacilli when they grow in the normal growth medium used- MRS (Man Rogasa Sharpe) produce significant amounts of lactic acid from the metabolism of glucose and a fully grown lactobacillus culture can drop the pH over night to ∼4.4-4.2.

*Clostridium difficile* strains are quite sensitive to acidic conditions and these conditions would not be encountered in the colon (normal pH ∼6.8) which is where *C. difficile* would normally be found.

Also the concentration of simple sugars like glucose would be negligible in the colon as monosaccharaides are more likely to be metabolised further up the GI tract.

We have developed a screening method to test lactobacilli strains and *C. difficile* when grown together in co-culture but without the drop in pH that would occur in the presence of high concentrations of sugar. We have eliminated the killing effect resulting from acid production by the lactobacilli therefore providing a method to isolate strains based on their ability to selectively target *C. difficle* through specific and unique mechanisms, not just generalised acid effects.

A novel co-culture medium containing minimized nutritional composition was developed. The new medium support limited growth of both *lactobacilli* and *C. difficile* cells when grown separately. The medium was rationally designed to simulate the lower gut environment in its composition so that cell growth was limited mainly by the amount of energy supply available. Furthermore, the medium was designed to have high buffering capacity to prevent a drop in pH during cell growth.

**Table 1: Comparison of de Man, Rogosa and Sharpe (MRS), Reinforced Clostridium Medium (RCM) and developed co-culture medium.**

| **Ingredient** | **MRS (g/l)** | **RCM (g/l)** | **Co-culture medium (g/l)** |
|---|---|---|---|
| Peptone | 10 | 10 | 2 |
| Meat Extract | 8 | 10 | 2 |
| Yeast extract | 4 | 3 | 1 |
| Glucose | 20 | 5 | 0.1 |
| Sodium acetate | 5 | 3 | 0.5 |
| Starch | - | 1 | - |
| Sodium chloride | - | 5 | 5 |
| Triammonium citrate | 2 | - | - |
| Magnesium sulphate | 0.2 | - | - |
| Manganese sulphate | 0.05 | - | - |
| Dipotassium phosphate | 2 | - | - |
| Tween 80 | 1 | - | - |
| L-cysteine HCl | - | 0.5 | 0.5 |
| Dihydrogen Sodium Phosphate | - | - | 3.7 |
| DiSodium Hydrogen Phospahte | - | - | 6.2 |
| pH | 6.2 | 6.8 | 6.8 |

### Example 1 Screening of Lactobacillus strains for anti-bacterial activity against Clostridium difficile

### Bacterial strains and growth conditions

*Lactobacillus* strains were maintained at -80°C in 40 % (v/v) glycerol and routinely cultured anaerobically at 37°C on de Man Rogosa Sharpe (MRS) agar (Difco, Beckton Dickinson & Co., New Jersey, USA) for 48 h or overnight in MRS broth. *C. difficile* strains EM304 (ribotype 027) and VPI 10463 (see Table 2 for details) were maintained at -80°C on micro-bank beads (Pro-Lab Diagnostics, Merseyside, UK) and cultured on Fastidious Anaerobic Agar (Lab M, Heywood, Lancashire, UK) supplemented with 7 % defibrinated horse blood (Cruinn Diagnostics, Dublin, Ireland) at 37°C for 3 days. Fresh cultures were grown overnight at 37°C in Reinforced Clostridium Medium (RCM) (Merck, Darmstadt, Germany) pre-boiled and cooled under anaerobic conditions. The lactobacilli and clostridia were grown in an anaerobic chamber (Don Whitley, West Yorkshire, UK) under an anoxic atmosphere (10 % H₂, 0 % O₂, 0 % N₂), unless otherwise stated.

**Table 2: Source and origin of critical bacterial strains either used or isolated in this study**

| Bacterial strain | Source | Origin |
|---|---|---|
| *Clostridium difficile* EM304 (ribotype 027) | ELDERMET Culture Collection¹ | elderly adult faeces |
| Clostridium difficile VPI 10463 (ATCC® 43255FZ™) | American Type Culture Collection² | human abdominal wound |
| *L. paracasei* strain APC1483 | APC Culture Collection¹ | Human faeces |
| L. gasseri APC 678 | APC Culture Collection¹ | human faeces |
| L. rhamnosus DPC 6111 | DPC Culture Collection¹ | human faeces |
| L. gasseri DPC 6112 | DPC Culture Collection¹ | healthy adult faeces |
| *L. gasseri* ATCC 33323 | American Type Culture Collection² | human faeces |

| | | |
|---|---|---|
| ¹Teagasc, Moorepark, Fermoy, Co. Cork, Ireland; ²Manassas, VA 20110, USA | | |

### Agar diffusion assay

Initially one thousand five hundred *Lactobacillus* isolates of food, human and animal origin were assessed for anti-bacterial activity against *C. difficile* EM304 using agar diffusion assays. Briefly, RCM agar was seeded with *C. difficile* and overnight cultures of lactobacilli, grown in MRS, were stabbed into the *C. difficile* seeded agar. Agar plates were incubated anaerobically at 37°C and assessed at 72 h for zones of inhibition against *C. difficile.* In addition *L. gasseri* APC 678 was assessed for bacteriocin activity against a range of target organisms as previously described (Rea *et al.,* 2010). The full list of target strains, together with their growth conditions are outlined in Table 3. Target organisms were chosen to test the effect of the novel lactobacillus strains on both potential gut pathogens and also gut commensals.

**Table 3: Target strains, growth medium and incubation conditions for well diffusion assays to detect bacteriocin production by strains of interest.**

| Target strain | Culture Collection No. | Incubation Conditions | Growth medium |
|---|---|---|---|
| Lactobacillus delbrueckii subsp. bulgaricus | LMG¹ 6901 | 37°C, anaerobic | MRS |
| Lactobacillus delbrueckii subsp. lactis | LMG 7942 | 37°C, anaerobic | MRS |
| Lactobacillus amylovorus | LMG 9496 | 37°C, anaerobic | MRS |
| Enterococcus saccharolyticus | LMG 11427 | 37°C, anaerobic | MRS |
| Enterococcus mundtii | LMG 10758 | 37°C, anaerobic | MRS |
| Listeria innocua | DPC² 3572 | 37°C, aerobic | BHI* |
| Enterococcus faecium | LMG 11423 | 37°C, anaerobic | MRS |
| Lactobacillus plantarum | LMG 6907 | 37°C, anaerobic | MRS |
| Micrococcus luteus | DPC 6275 | 30°C, aerobic | BHI |
| Lactobacillus acidophilus | LMG 9433 | 37°C, anaerobic | MRS |
| Staphylococcus aureus | DPC 5246 | 37°C, aerobic | BHI |
| Salmonella typhimurium | DPC 6048 | 37°C, aerobic | BHI |
| Lactobacillus agilis | LMG 9186 | 37°C, anaerobic | MRS |
| Lactobacillus rhamnosus GG | ATCC³ 53103 | 37°C, anaerobic | MRS |
| Lactobacillus casei | LMG 6904 | 37°C, anaerobic | MRS |
| Lactobacillus crispatus | LMG 9479 | 37°C, anaerobic | MRS |
| Lactobacillus fermentum | LMG 6902 | 37°C, anaerobic | MRS |

| | | | |
|---|---|---|---|
| ¹LMG Culture Collection, B-9000 Gent, Belgium; ²DPC Culture Collection, Teagasc, Moorepark, Fermoy, Co. Cork, Ireland; ³American Type Culture Collection, Manassas, VA 20110, USA. *BHI: Brain heart infusion medium, Merck, Darmstadt, Germany. | | | |

Initial screening for the production of antibacterial compounds by lactobacilli against *C. difficile* using the antagonistic agar assay failed to reveal zones of inhibition. Therefore, a low nutrient media (MGM) - the novel co-culture medium - was developed which represented more closely the low concentration of simple carbohydrates in the human gut.

### Co-culture broth

This media allowed growth of both *Lactobacillus* and *C. difficile* strains by ∼1 to 2 logs over a 24h period. Due to the buffering capacity of the medium, the pH was maintained at near neutral (∼pH 6.5) following growth for 24 h, eliminating concerns relating to the reduction of *C. difficile* merely as a result of acid production.

Fifty eight *Lactobacillus* strains (Table 4) from human and animal origin were selected for further screening using a co-culture method, with C. *difficile* EM 304 (ribotype 027) (Rea *et al.,* 2012b) as the target strain. A minimal growth medium (MGM) - the co-culture medium - was developed to represent the nutrient limited content of the human gut and to allow co-culturing of *Lactobacillus* and *Clostridium* strains. The media composition (g L⁻¹) was: meat extract, 2 g; peptone, 2 g; yeast extract, 1 g; NaCl, 5 g; sodium acetate, 0.5 g; L-cysteine hydrochloride, 0.5 g; glucose, 0.1 g; NaH₂PO₄.H₂0, 3.7 g; Na₂HPO₄.7H₂O, 6.2 g; pH 6.8 (± 0.2). Following overnight growth, 1 ml of *C. difficile* EM304 and each *Lactobacillus* strain were centrifuged at 14,000 x g. Pelleted cells were washed once in phosphate buffered saline (PBS) under anaerobic conditions and resuspended in fresh PBS. The MGM was inoculated with a test strain of *Lactobacillus* and *C. difficile* EM304 at 10⁶ CFU ml⁻¹. The tubes were incubated at 37°C for 24 h. Survival of *C. difficile* was determined by plating onto Brazier's cefoxitin cycloserine and egg yolk agar (CCEY) (Lab M), and *Lactobacillus* counts were determined by plating onto MRS agar. Plates were incubated anaerobically at 37°C for 2-3 days and the anti-bacterial ability was determined as a reduction in *C. difficile* counts compared to the control in the absence of *Lactobacillus.*

**Table 4: Lactobacilli screened for anti-Clostridium difficile activity.**

| Lactobacillus spp. | No of strains tested | Origin |
|---|---|---|
| Lactobacillus gasseri | 10 | human faeces |
| Lactobacillus casei | 7 | human faeces |
| Lactobacillus paracasei | 4 | human faeces |
| Lactobacillus salivarius | 5 | 4 human faeces; 1 pig faeces |
| Lactobacillus rhamnosus | 6 | human faeces |
| Lactobacillus brevis | 4 | 2 human faeces; 1 cow faeces; 1 silage |
| Lactobacillus mucosae | 3 | cow faeces |
| Lactobacillus plantarum | 4 | cow faeces; hand wash water; milking yard water |
| Lactobacillus parabuchneri/kefir | 2 | 1 pig caecum; 1 milk |
| Lactobacillus ruminis | 2 | human faeces |
| Lactobacillus reuteri | 2 | 1 human faeces; 1 pig faeces |
| Lactobacillus acidophilus | 1 | human faeces |
| Lactobacillus murinus | 1 | pig caecum |
| Uncharacterised lactobacilli | 7 | human faeces |

The co-culture assay showed that 4/58 lactobacilli tested *(L. gasseri* APC 678, *L. rhamnosus* DPC 6111, *L. gasseri* DPC 6112 and *L. paracasei* APC 1483) had the ability to reduce the growth/survival of C. *difficile in vitro* (Figure 1). Of these, the two best performing strains were *L. gasseri* APC 678 and *L. rhamnosus* DPC 6111. In contrast, the well characterised *L. gasseri* ATCC 33323 strain did not show any inhibitory effect.

### Example 2 - Identity of L. gasseri APC678 was confirmed by BLAST analysis of the 16S rRNA gene region.

### Method

16s rRNA gene sequencing (16S) was performed to identify *L. gasseri* APC678. Briefly, total DNA was isolated from the strains using 100 µl of Extraction Solution and 25 µl of Tissue Preparation solution (Sigma-Aldrich, XNAT2 Kit). The samples were incubated for 5 minutes at room temperature followed by 2 h at 95 °C. 100 µl of Neutralization Solution (Sigma-Aldrich, XNAT2 kit) was then added. DNA solution was quantified using a Nanodrop spectrophotometer and stored at 4°C. PCR was performed using the 16S primers. The primer pairs used for identification of the both strain were 16S Forward 5'- CTG ATC TCG AGG GCG GTG TGT ACA AGG -3' and 16S Reverse 5'- CTG ATG AAT TCG AGA CAC GGT CCA GAC TCC-3'. The cycling conditions were 94°C for 4 min (1 cycle), 94°C for 45 sec, 56°C for 45 sec, 72°C for 45 sec (30 cycles). The PCR reaction contained 2 µl (100 ng) of DNA, PCR mix (Sigma-Aldrich, Red Taq), 0.025 nM 16S L and R primer (MWG Biotech, Germany). The PCR reactions were performed on an Eppendorf thermocycler. The PCR products were run alongside a molecular weight marker (100 bp Ladder, Roche) on a 2 % agarose EtBr stained gel in TAE, to determine the 16S profile. PCR products were purified using the Promega Wizard PCR purification kit. The purified PCR products were sequenced at Beckman Coulter Genomics (UK) using the primer sequences (above) for the 16S rRNA gene region. Sequence data was then searched against the NCBI nucleotide database to determine the identity of the strain by nucleotide homology. The resultant DNA sequence data was subjected to the NCBI standard nucleotide-to-nucleotide homology BLAST search engine (http://www.ncbi.nm.nih.gov/BLAST/) to identify the nearest match to the sequence.

### Results

Identity *of L. gasseri* APC678 was confirmed by BLAST analysis of the 16S rRNA gene region.

**Table 5: Blast results of the 16S rRNA gene region of L. gasseri APC678.**

| **Sample ID** | **Accession #** | **Closest Match on NCBI BLAST** | **Identities** | **% match** | **bp** | **Query Coverage** | **E value** |
|---|---|---|---|---|---|---|---|
| *L. gasseri* APC678 | KU710510.1 | *Lactobacillus gasseri* strain LG202 | 1024/1028 | 99% | 1026 | 100% | 0% |

**Table 6: Sequence of the 16S rRNA gene region of L. gasseri APC678.**

| 16s rRNA sequence of *L. gasseri* APC678 (1026 nt): |
|---|
| TAGCACGTGTGTAGCCCAGGTCATAAGGGGCATGATGACTTGACGTCATCCCCACCT TCCTCCGGTTTGTCACCGGCAGTCTCATTAGAGTGCCCAACTTAATGATGGCAACTA ATGACAAGGGTTGCGCTCGTTGCGGGACTTAACCCAACATCTCACGACACGAGCTG ACGACAGCCATGCACCACCTGTCTCAGCGTCCCCGAAGGAACACCTAATCTCTTAG GTTTGCACTGGATGTCAAGACCTGGTAAGGTTCTTCGCGTTGCTTCGAATTAAACCA CATGCTCCACCGCTTGTGCGGGCCCCCGTCAATTCCTTTGAGTTTCAACCTTGCGGTC GTACTCCCCAGGCGGAGTGCTTAATGCGTTAGCTGCAGCACTGAGAGGCGGAAACC TCCCAACACTTAGCACTCATCGTTTACGGCATGGACTACCAGGGTATCTAATCCTGT TCGCTACCCATGCTTTCGAGCCTCAGCGTCAGTTGCAGACCAGAGAGCCGCCTTCGC CACTGGTGTTCTTCCATATATCTACGCATTCCACCGCTACACATGGAGTTCCACTCTC CTCTTCTGCACTCAAGTTCAACAGTTTCTGATGCAATTCTCCGGTTGAGCCGAAGGC TTTCACATCAGACTTATTGAACCGCCTGCACTCGCTTTACGCCCAATAAATCCGGAC AACGCTTGCCACCTACGTATTACCGCGGCTGCTGGCACGTAGTTAGCCGTGACTTTC TAAGTAATTACCGTCAAATAAAGGCCAGTTACTACCTCTATCTTTCTTCACTACCAA CAGAGCTTTACGAGCCGAAACCCTTCTTCACTCACGCGGCGTTGCTCCATCAGACTT GCGTCCATTGTGGAAGATTCCCTACTGCTGCCTCCCGTAGGAGTCTGGACCGTGTC |

### Example 3 - Screening for survival through gastrointestinal transit

Among the important traits required for live microbes intended for use in the gastrointestinal tract (GIT) is the ability to survive the acidic conditions of the stomach and the presence of bile in the upper small intestine. The ability of the selected bacterial strains to survive a simulated gastric environment was assessed. Briefly, MRS broth was inoculated at 1 % with the *Lactobacillus* strains and incubated anaerobically at 37°C for 16 h. One ml of cells was centrifuged at 14,000 x g, washed in PBS and re-centrifuged. The cells were then resuspended in PBS or 10 % reconstituted skim milk (RSM). A suspension of 10⁸ CFU ml⁻¹ *Lactobacillus* was suspended in artificial gastric juice with the following composition: NaCl, 125 mmol L⁻¹; KCl, 7 mmol L⁻¹; NaHCO₃, 45 mmol L⁻¹ and pepsin, 3 g L⁻¹. The final pH was adjusted with HCl to pH 2 and pH 3 and with NaOH to pH 7. The bacterial suspensions were incubated at 37°C with agitation (200 rev min⁻¹) to stimulate peristalsis. Aliquots were taken for enumeration of viability after 0, 90 and 180 min.

Following 180 min suspension in simulated gastric juice, the cells were suspended in simulated intestinal fluid, which was prepared with 0.1 % (w/v) pancreatin (Sigma) and 0.15 % oxgall bile salts (Difco), in water adjusted to pH 8.0 with NaOH, for a further 180 min. The suspensions were incubated at 37°C and samples taken for total viability after 90 and 180 min. Viability was assessed by plating serial dilutions on MRS agar and incubating at 37°C for 48 h. Survival was expressed as log reduction from 0 h.

We have demonstrated the survival of *L. gasseri* APC 678 and *L. rhamnosus* DPC 6111 in a simulated GIT environment (Table 7). Both strains, when suspended in PBS before being added to simulated gastric juice at pH 3 were very stable. However, *L. gasseri* APC 678 was the more stable of the two at pH 2, showing a 1.5 log reduction in total viable counts compared to 3.5 log reduction for *L. rhamnosus* DPC 6111. Viable counts indicated that neither strain survived the subsequent 3 h incubation in simulated ileal juice. However, if the strains were suspended in 10 % RSM prior to treatment with gastric/ileal juice, their survival was markedly improved both in simulated gastric juice at both pH 2 and 3 and also after a further 3 h incubation in ileal juice. In 10 % RSM *L. rhamnosus* DPC 6111 was more sensitive to the conditions of the stomach and ileum than *L. gasseri* APC 678, showing ∼4.5 log reduction at the end of the incubation period compared with a reduction of 2.8 logs for *L. gasseri* APC 678.

**Table 7: Survival of lactobacilli during simulated gastrointestinal tract transit. The effect of initial suspension medium (PBS or RSM) on the subsequent survival of L. gasseri APC 678 and L. rhamnosus DPC 6111 in simulated gastric juice followed by simulated ileal juice.**

| | | Log reduction (CFU/ml) after 3 h in simulated gastric juice | | Log reduction (CFU/ml) after a further 3 h in simulated ileal juice* | |
|---|---|---|---|---|---|
| Suspension medium | pH | *L. gasseri* APC 678 | *L. rhamnosus* DPC 6111 | *L. gasseri* APC 678 | *L. rhamnosus* DPC 6111 |
| PBS | 7.0 | 0.16 | 0.21 | 0.52 | 0.43 |
| | 3.0 | 0.96 | 1.01 | 2.19 | 1.78 |
| | 2.0 | 1.52 | 3.53 | 8.00 | 8.00 |
| RSM | 7.0 | 0.84 | 0.49 | 0.52 | 0.13 |
| | 3.0 | 1.92 | 0.91 | 1.89 | 2.47 |
| | 2.0 | 1.91 | 1.49 | 2.82 | 4.39 |

| | | | | | |
|---|---|---|---|---|---|
| *Cells were transferred to simulated ileal juice following 3 h incubation in gastric juice and incubated for an additional 3 h. | | | | | |

These results demonstrated the suitability of these strains for consumption due to their inherent ability to survive gastric transit in an *in vitro* model system.

### Example 4 - In vivo assessment of strains in C. difficile carriage mouse model

The ability of *L*. *gasseri* APC 678 and *L. rhamnosus* DPC 6111 to reduce C. *difficile* in *vivo* in a murine model was investigated. This model serves two purposes. It acts as a model of diversity reduction post-antibiotic use in addition to being a model of c. *difficile* colonisation and disease. In addition, the well characterised strain *L. gasseri* ATCC 33323 (Azcarate-Peril *et al.,* 2008), which did not show any inhibition of C. *difficile* using the afore-mentioned *in vitro* assays, was selected as a negative control for the animal study.

### Mouse model

### Materials and Methods

All procedures involving animals were approved by the UCC Animal Experimentation Ethics Committee (#2011/17). For the C. *difficile* model, 40 female C57BL/6 mice (7 weeks old) were obtained from Harlan Laboratories UK (Bicester, Oxfordshire, UK). All mice used in the experiment were housed in groups of 5 animals per cage under the same conditions. Food, water, bedding and cages were autoclaved before use.

### Antibiotic administration

All mice were made susceptible to C. *difficile* infection by altering the gut microbiota using a previously described protocol (Chen *et al.,* 2008). Briefly, an antibiotic mixture comprising of kanamycin (0.4 mg mL⁻¹), gentamicin (0.035 mg mL⁻¹), colistin (850 U mL⁻¹), metronidazole (0.215 mg L⁻¹) and vancomycin (0.045 mg mL⁻¹) was prepared in water (all antibiotics were purchased from Sigma Aldrich, Ireland). This corresponds to the approximate daily dose for each antibiotic as follows kanamycin (40 mg kg⁻¹), gentamicin (3.5 mg kg⁻¹), colistin (4.2 mg kg⁻¹), metronidazole (21.5 mg kg⁻¹) and vancomycin (4.5 mg kg⁻¹). The concentrations of antibiotics in the water were calculated based on the average weight of the animals and expected daily water consumption of the mice. All mice received the antibiotic cocktail in water for 3 days, followed by 2 days of water without antibiotics. All mice received a single dose of clindamycin 10 mg kg-¹ intra-peritoneally 1 day before *C. difficile* challenge.

### Preparation of bacterial cultures

Adhering to strict anaerobic conditions, *C. difficile* strain VPI 10463 was grown overnight in RCM. Bacterial cells were collected by centrifugation at 4,050 x g for 5 minutes, washed once in PBS and resuspended in PBS to achieve a preparation of 5 x 10⁵ CFU per mouse. *Lactobacillus* strains for each group - *L. gasseri* APC 678, *L. rhamnosus* DPC 6111 and *L. gasseri* ATCC 33323 (see Table 4 for details) were prepared by growing the strains overnight in MRS broth under anaerobic conditions. Cells were collected by centrifugation at 4,050 x g for 5 minutes, washed once in saline solution and resuspended in 10 % (w/v) reconstituted skim milk (RSM) to achieve 1 x 10⁸ CFU per mouse in 100 µl RSM. The control group were fed 10 % RSM only. At the start of the experiment all mice (10/group) received an individual inoculum of *C. difficile (5* x 10⁵ CFU/mouse). Five hours later 100 µl of the target strain (equivalent to 1 x 10⁸ CFU) or RSM (control group) was administered by oral gavage daily for 7 days.

### Sample collection and C. difficile counts

Prior to commencement of the trial and before antibiotic treatment, faecal samples were collected from all animals and plated on CCEY agar to confirm that the mice were *C. difficile-*free. Subsequently, faecal pellets were collected at 24 h, 4 days and 7 days post-infection with *C. difficile* and stored anaerobically before being assessed for viable *C. difficile* (CFU g⁻¹ faeces). At the end of the trial the mice were sacrificed and total numbers of C. *difficile* per colon were counted (CFU colon⁻¹). *C. difficile* survival was determined by culturing anaerobically at 37°C on CCEY agar for 48 h. *C. difficile* colonies were confirmed using the Oxoid *C. difficile* test kit (Oxoid, Basingstoke, UK). At the time of culling caecal contents were collected for compositional sequencing from each individual mouse, snap frozen and stored at -80°C until required.

### Microbial DNA extraction, 16s rRNA amplification and Illumina MiSeq sequencing

Total metagenomic DNA was extracted for each mouse caecum, following thawing at 4°C, with the QIAamp DNA Stool Mini Kit (Qiagen, Hilden, Germany) with an additional bead beating step (Murphy *et al.,* 2010). DNA was quantified using the Nanodrop 1000 spectrophotometer (Thermo Scientific, Ireland). Initially the template DNA was amplified using primers specific to the V3-V4 region of the 16s rRNA gene which also allowed for the Illumina overhang adaptor, where the forward (5'TCGTCGGCAGCGTCAGATGTGTATAAGAGACAGCCTACGGGNGGCWGCAG) and reverse primers (5'GTCTCGTGGGCTCGGAGATGTGTATAAGAGACAGGACTACHVGGGTATCTAATCC )
were used. Each PCR reaction contained 2.5 µl DNA template (5 ng), 5 µl forward primer (1 µM), 5 µl reverse primer (1 µM) (Sigma) and 12.5 µl Kapa HiFi Hotstart Readymix (2X) (Anachem, Dublin, Ireland). The template DNA was amplified under the following PCR conditions for a total of 25 cycles: 95°C for 3 minutes and 30 seconds respectively (initialization and denaturation), 55°C for 30 seconds (annealing) and 72°C for 30 seconds (elongation), followed by a final elongation step of 5 minutes. PCR products were visualised using gel electrophoresis (1X TAE buffer, 1.5 % agarose gel, 100 V) post PCR reaction. Successful amplicons were cleaned using the AMpure XP purification system (Labplan, Dublin, Ireland). A second PCR reaction was completed using the previously amplified and purified DNA as the template. Two indexing primers (Illumina Nextera XT indexing primers, Illumina, Sweden) were used per sample to allow all samples to be pooled, sequenced on one flow cell and subsequently identified bioinformatically. Each reaction contained 25 µl Kapa HiFi HotStart ReadyMix (2X), 5 µl template DNA, 5 µl index primer 1 (N7xx), 5 µl index primer 2 (S5xx) and 10 µl PCR grade water. PCR conditions were the same as previously described with the samples undergoing just 8 cycles instead of 25. PCR products then underwent the same electrophoresis and cleaning protocols as described above. Samples were quantified using the Qubit 2.0 fluorometer (Invitrogen, Carlsbad, CA, USA) in conjunction with the broad range DNA quantification assay kit (Biosciences, Dublin, Ireland). All samples were pooled to an eqimolar concentration. Quality of the pool was determined by running on the Agilent Bioanalyser prior to sequencing. The sample pool was then denatured with 0.2 M NaOH, diluted to 4 pM and combined with 10 % (v/v) denatured 4 pM PhiX. Samples were sequenced on the MiSeq sequencing platform (Teagasc Sequencing Centre, Moorepark, Fermoy, Co. Cork, Ireland) using a 2300 cycle V3 Kit following protocols outlined by Illumina.

### Bioinformatic analysis

Raw Illumina 300 base pair paired-end sequence reads were merged using Flash (Magoc and Salzberg, 2011) and quality checked using the split libraries script from the Qiime package (Caporaso *et al.,* 2010). Reads were then clustered into operational taxonomical units (OTUs) and chimeras removed with the 64-bit version of USEARCH (Edgar, 2010). Subsequently OTUs were aligned and a phylogenetic tree generated within Qiime. Taxonomical assignments were reached using the SILVA 16S specific database (version 111) (Quast *et al.,* 2013). PICRUSt (phylogenetic investigation of communities by reconstruction of unobserved states) analysis was performed on the OTU tables to infer function (Langille *et al.,* 2013). Alpha and beta diversity analysis was also implemented within Qiime. Principal coordinate analysis (PCoA) plots were then visualised using EMPeror v0.9.3-dev (Vazquez-Baeza *et al.,* 2013).

### Statistical analysis

Non-parametric Mann-Whitney statistical analysis was applied on MiniTab (Version 15; faecal and colon culture data) and SPSS (PASW Statistics version 18; caecal microbiota compositional data) statistical packages, to assess whether differences in *C. difficile* shedding, microbiota composition and diversity between the control and probiotic-fed groups were significant. Statistical significance was accepted at p<0.05, adjusted for ties, where the null hypothesis was rejected.

### Results

The reduction of *C. difficile* shedding in the faeces, the reduction of total viable *C. difficile* in the mouse colon and changes in the microbiota composition of the mouse caecum were assessed. Mice were infected with ∼5 x 10⁵ CFU of *C. difficile* and at day 1 the mean *C. difficile* counts were >10⁷/g of faeces in all groups, which compares well with *C. difficile* counts in murine studies where the animals received clindamycin or metronidazole prior to infection with *C. difficile* (Schubert *et al.,* 2015b). There was no significant reduction in the numbers of *C. difficile* shed in the faeces between the control group (fed RSM only) and the lactobacillus-fed mice after 24 h (Figure 2a). However, after 4 and 7 days, *L. gasseri* APC 678 significantly reduced *C. difficile* faecal shedding (p<0.05) compared to the control mice, while there was no significant reduction in *C. difficile* in the faeces of those mice receiving either *L. rhamnosus* DPC 6111 or *L. gasseri* ATCC 33323 compared to the control mice (Figure 2b and 2c). It was also noted that, by day 7, both *L. gasseri* APC 678 and *L. gasseri* ATCC 33323 significantly reduced the numbers of *C. difficile* that had adhered to the colon (p=0.003 and p= 0,014 respectively); Figure 2d).

### Effects of the chosen bacteria on the gut microbiota

Significant effects of the chosen bacteria on the gut microbiota were seen. These effects were strain specific and surprising.

Diversity Indices higher in APC687 fed mice compared to controls and other strains tested.

Following total metagenomic DNA extraction of the caecal contents, V3-V4 16S rRNA gene amplicons were generated and sequenced using the Illumina MiSeq. Following quality filtering, 4,985,283 sequence reads remained. Diversity, richness and coverage estimations were calculated for each data set (Table 6), all of which indicated good sample richness throughout and the presence of a diverse microbiota. Interestingly, the Simpson and Shannon diversity metrics were significantly higher in the *L. gasseri* APC 678-fed mice compared to the control mice, and all alpha diversity indices tested were significantly higher in the *L. gasseri* APC 678-fed mice compared to the those fed *L. gasseri* ATCC33323 or *L. rhamnosus* DPC 6111, indicating that *L. gasseri* APC 678 had a greater impact on diversity than the other probiotics tested. Beta-diversity was estimated using distance matrices built from unweighted Unifrac distances and, subsequently, principal co-ordinate analysis (PCoA) was performed on the distance matrices. The different groups cluster on the basis of strain administrated.

*Differences in effect on abundance of specific phyla seen between strains* Sequence analysis revealed that the microbiota was comprised of 7 main phyla (Table 7, and Table 8), with Firmicutes and Bacteroidetes dominating, and relative abundance corresponding to 28-55 % and 43-71%, respectively. Unlike the group fed *L. gasseri* APC 678, where no significant change in the abundance of Firmicutes or Bacteroidetes relative to controls was observed, the groups fed *L*. *rhamnosus* DPC 6111 or *L. gasseri* ATCC 33323 showed a significant decrease in the relative abundance of Firmicutes (p=0.002 and 0.019, respectively) and a significant increase in Bacteroidetes (p=0.002 and 0.023, respectively) relative to the control. The relative abundance of the Phylum Proteobacteria significantly decreased in the mice fed APC 678 or DPC 6111 relative to the control mice or the animals fed ATCC 33323 which in fact showed an increase in Proteobacteria relative to the control. This change was mirrored in the significant reduction of the relative abundance of the genera *Escherichia*/*Shigella* in the groups fed *L. gasseri* APC 678 and *L. rhamnosus* DPC6111. Elevated Proteobacteria are normally associated with antibiotic use and are elevated in patients with CDI (Cotter *et al* and Milani et al 2016) indicating that the abundance profiles seen post feeding APC678 or with *L. rhamnosus* DPC6111 are more consistent with a healthy diversity profile at the phylum level.

Differences in effect on abundance of specific bacterial families and genera within phyla seen on administration of different bacterial strains.

A diverse range of microbial families (Table 9) and genera (Table 10) were also detected across the 4 feeding groups. A number of statistical differences were found in OTUs at family and genus level (Table 9).

The relative abundance of the genus Alistipes significantly increased in mice fed all *Lactobacillus* strains. The increase was highest in animals *L. gasseri* ATCC 33323-fed group. A study by Schubert et al (2015) showed that in a murine model Alistipes protected against C. *difficile* colonisation and the relative abundance of this genus has been shown to be decreased in CDI patients compared to non CDI patients not in receipt of antibiotics (Milani *et al.,* 2016; Schubert *et al.,* 2015a). The *L. gasseri* ATCC33323-fed group did not work in the *in vivo* mouse model however. Further interrogation of the diversity indices did indicate that *L. gasseri* APC 678 and to a lesser extent *L. rhamnosus* DPC6111 had multiple effects on different bacterial families all of which have been linked to effects in CDI.

The relative abundance of the genera *Escherichia*/*Shigella* was significantly reduced in the groups fed *L. gasseri* APC 678 and *L. rhamnosus* DPC6111. The decrease in the *L. gasseri* ATCC 33233-fed group was not significant. The reduction in the genera Escherichia/Shigella would be considered a positive attribute and the relative abundance of these genera was shown to increase in CDI patients in a study where the faecal microbiota of 25 CDI positive patients was compared to a control group (n=30) who were CDI negative and where not exposed to antibiotics (Milani *et al.,* 2016).

The relative abundance of Rikenellaceae RC9 gut group significantly increased in all lactobacillus-fed groups compared to the control group with the largest increase associated with *L. gasseri* APC 678-fed group. The relative abundance of RC9 gut group was shown to be decreased in a cohort of CDI patients relative to that group who were CDI negative and had not been exposed to antibiotics (Milani *et al.,* 2016).The relative abundance of *Peptostreptococcaceae Incertae Sedis* were significantly reduced in all Lactobacillus-fed groups with both *L. gasseri* strains showing the largest decrease. This family encompasses *C. difficile* which has now been reclassified as *Peptoclostridium difficile (Yutin and Galperin, 2013)*(6).

Strain specific effects were seen for *L. gasseri* APC 678 and *L. rhamnosus* DPC 6111 which are associated with the presence or absence of non-communicable disease.

The relative abundance of *Roseburia* significantly increased in all Lactobacillus-fed groups but the largest increase was seen in the *L. gasseri* APC 678-fed group. Roseburia are associated with the production of short chain fatty acid production in the gut. SCFA are known to have anti-inflammatory, anti-tumorigenic, and antimicrobial activity and can be metabolised by host epithelial cells in the colon (Rios-Covian *et al.,* 2016).

The relative abundance of *Oscillibacter* significantly increased in the groups fed both *L. gasseri* APC 678 or *L. rhamnosus* DPC 6111 but not in the *L. gasseri* ATCC 33323-fed group. Some species of *Oscillibacter* are associated with the production of SCFA producing predominantly valerate (Iino *et al.,* 2007).

**Table 8: Alpha diversity indices for sequencing coverage and diversity of microbiota of caecum samples at Day 7 from control and the mice fed the test strains.**

| Alpha diversity index | Control | *L. gasseri* APC 678 | *L. rhamnosus* DPC 6111 | *L. gasseri* ATCC 33323 |
|---|---|---|---|---|
| Chao1 richness estimate | 210 | 272^{a} | 194 | 168 |
| Simpson diversity index | 0.92 | 0.94*^{, a} | 0.92 | 0.89 |
| Shannon diversity index | 4.77 | 5.45*^{, a} | 4.69 | 4.41 |
| PD whole tree | 11.69 | 14.06^{a} | 11.06 | 9.73 |
| Number of observed species | 197 | 259^{a} | 182 | 153 |

| | | | | |
|---|---|---|---|---|
| *significantly different compared to the control; ^{a}significantly different compared to *L. rhamnosus* DPC 6111 and *L. gasseri* ATCC 33323; non-parametric Mann-Whitney test was used to estimate the relationship between the groups; statistical significance was accepted at p<0.05. | | | | |

**Table 9: Relative abundance (%) of bacterial phyla in the caecum at Day 7 of the control and probiotic-fed mice (Lactobacillus gasseri APC 678, Lactobacillus rhamnosus DPC 6111 and Lactobacillus gasseri ATCC 33323).**

| Group | Control | APC 678 | DPC 6111 | ATCC 33323 |
|---|---|---|---|---|
| | relative abundance (%) | | | |
| Firmicutes | 54.51 | 37.22 | 28.44* | 31.29* |
| Bacteroidetes | 43.17 | 62.24 | 70.62* | 66.25* |
| Verrucomicrobia | 1.53 | 0.08 | 0.35 | 0.93 |
| Proteobacteria | 0.47 | 0.19* | 0.09* | 1.29 |
| Actinobacteria | 0.26 | 0.18 | 0.19 | 0.13* |
| Tenericutes | 0.01 | 0.03 | 0.26 | 0.10 |
| Cyanobacteria | 0.00 | 0.00 | 0.00 | 0.00 |
| Other | 0.04 | 0.05 | 0.05 | 0.02 |

| | | | | |
|---|---|---|---|---|
| *significantly different compared to the control; significance was determined by p<0.05. | | | | |

**Table 10: Relative abundance (%) at bacterial phylum, family and genus level in the caecum at Day 7 of the control and test mice (Lactobacillus gasseri APC 678, Lactobacillus rhamnosus DPC 6111 and Lactobacillus gasseri ATCC 33323). Only phyla, families and genera with significant differences compared to the control mice are represented.**

| Group | Control | APC 678 | DPC 6111 | ATCC 33323 |
|---|---|---|---|---|
| | Relative abundance (%) | | | |
| Phylum: | | | | |
| Firmicutes | 54.51 | 37.22 | 28.44* | 31.29* |
| Bacteroidetes | 43.17 | 62.24 | 70.62* | 66.25* |
| Proteobacteria | 0.47 | 0.19* | 0.09* | 1.29 |
| Actinobacteria | 0.26 | 0.18 | 0.19 | 0.13* |
| Family: | | | | |
| Lachnospiraceae | 40.35 | 27.46 | 20.14* | 23.60* |
| S24-7 | 24.10 | 31.18 | 40.72* | 23.21 |
| Bacteroidaceae | 9.29 | 21.60 | 18.61 | 28.21* |
| uncultured Clostridiales | 2.01 | 0.64 | 0.21* | 0.58* |
| Rikenellaceae | 1.86 | 7.31* | 6.52* | 8.11* |
| Erysipelotrichaceae | 1.80 | 0.62* | 0.83* | 0.95* |
| Peptostreptococcaceae | 0.60 | 0.08* | 0.02* | 0.05* |
| Alcaligenaceae | 0.29 | 0.09* | 0.069* | 1.24 |
| Enterobacteriaceae | 0.18 | 0.01* | 0.02* | 0.06 |
| Bifidobacteriaceae | 0.13 | 0.04* | 0.02* | 0.07* |
| Enterococcaceae | 0.03 | 0.02 | 0.01* | 0.01 |
| Peptococcaceae | 0.00 | 0.04* | 0.02 | 0.07 |
| Prevotellaceae | 0.00 | 0.53 | 0.56* | 0.00 |
| Xanthomonadaceae | 0.00 | 0.06* | 0.00 | 0.00 |
| Genus: | | | | |
| uncultured Lachnospiraceae | 29.99 | 20.93 | 14.78* | 18.00* |
| uncultured S24-7 | 24.10 | 31.18 | 40.72* | 23.21 |
| Bacteroides | 9.29 | 21.60 | 18.61 | 28.21* |
| *Ruminococcaceae* Incertae Sedis | 5.36 | 2.19* | 1.44* | 2.01* |
| uncultured Clostridiales | 2.01 | 0.64 | 0.21* | 0.58* |
| Alistipes | 1.41 | 3.40* | 4.95* | 6.03* |
| uncultured Ruminococcaceae | 1.08 | 2.03* | 0.77 | 1.31 |
| Peptostreptococcaceae Incertae Sedis | 0.60 | 0.08* | 0.02* | 0.05* |
| Anaerotruncus | 0.60 | 1.09 | 1.85* | 0.76 |
| *Rikenellaceae* RC9 gut group | 0.45 | 3.90* | 1.57* | 2.08 |
| Oscillibacter | 0.36 | 1.15* | 1.81* | 0.79 |
| Parasutterella | 0.29 | 0.09* | 0.07* | 1.24 |
| Flavonifractor | 0.25 | 0.01* | 0.01* | 0.09 |
| Roseburia | 0.21 | 0.90* | 0.21 | 0.29 |
| Escherichia-Shigella | 0.18 | 0.01* | 0.02* | 0.06 |
| uncultured Erysipelotrichaceae | 0.14 | 0.04* | 0.05* | 0.04* |
| Bifidobacterium | 0.13 | 0.04* | 0.02* | 0.07* |
| Enterococcus | 0.03 | 0.02 | 0.007* | 0.01 |
| uncultured Peptococcaceae | 0.00 | 0.03* | 0.01 | 0.07 |
| Prevotella | 0.00 | 0.53 | 0.56* | 0.00 |
| Hydrogenoanaerobacterium | 0.00 | 0.004* | 0.00 | 0.00 |
| Stenotrophomonas | 0.00 | 0.06* | 0.00 | 0.00 |

| | | | | |
|---|---|---|---|---|
| *significantly different compared to the control; significance was determined by p<0.05, where the null hypothesis was rejected. | | | | |

**Table 11: Relative abundance (%) of bacterial families in the caecum at Day 7 of the control and test mice (fed Lactobacillus gasseri APC 678, Lactobacillus rhamnosus DPC 6111 and Lactobacillus gasseri ATCC 33323).**

| Group | Control | APC 678 | DPC 6111 | ATCC 33323 |
|---|---|---|---|---|
| Lachnospiraceae | 40.346 | 27.461 | 20.141* | 23.600* |
| S24-7 | 24.104 | 31.182 | 40.720* | 23.214 |
| Bacteroidaceae | 9.289 | 21.603 | 18.614 | 28.214* |
| Porphyromonadaceae | 7.918 | 1.611 | 4.213 | 6.710 |
| Ruminococcaceae | 7.743 | 6.576 | 5.874 | 4.957 |
| uncultured Clostridiales | 2.008 | 0.639 | 0.213* | 0.582* |
| Rikenellaceae | 1.859 | 7.314* | 6.515* | 8.112* |
| Lactobacillaceae | 1.835 | 1.078 | 1.259 | 1.036 |
| Erysipelotrichaceae | 1.804 | 0.616* | 0.829* | 0.948* |
| Verrucomicrobiaceae | 1.531 | 0.084 | 0.354 | 0.928 |
| Peptostreptococcaceae | 0.603 | 0.078* | 0.019* | 0.050* |
| Alcaligenaceae | 0.286 | 0.085* | 0.069* | 1.235 |
| Enterobacteriaceae | 0.177 | 0.010* | 0.017* | 0.055 |
| Coriobacteriaceae | 0.130 | 0.140 | 0.167 | 0.063 |
| Bifidobacteriaceae | 0.126 | 0.039* | 0.024* | 0.065* |
| Christensenellaceae | 0.103 | 0.375 | 0.000 | 0.012 |
| Enterococcaceae | 0.030 | 0.023 | 0.007* | 0.013 |
| Clostridiales Family XIII Incertae Sedis | 0.019 | 0.028 | 0.029 | 0.019 |
| Clostridiaceae | 0.016 | 0.000 | 0.002 | 0.001 |
| Anaeroplasmataceae | 0.013 | 0.033 | 0.258 | 0.097 |
| Oxalobacteraceae | 0.005 | 0.000 | 0.000 | 0.000 |
| Peptococcaceae | 0.004 | 0.041* | 0.020 | 0.066 |
| Rhodospirillaceae | 0.002 | 0.008 | 0.000 | 0.000 |
| Staphylococcaceae | 0.002 | 0.288 | 0.045 | 0.000 |
| Streptococcaceae | 0.001 | 0.001 | 0.000 | 0.000 |
| *Rickettsiales* mitochondria | 0.001 | 0.000 | 0.000 | 0.000 |
| Cyanobacteria 4C0d-2 uncultured | 0.001 | 0.000 | 0.000 | 0.000 |
| Moraxellaceae | 0.001 | 0.005 | 0.000 | 0.000 |
| Planococcaceae | 0.000 | 0.012 | 0.000 | 0.000 |
| Prevotellaceae | 0.000 | 0.533 | 0.561* | 0.001 |
| *Bacteroidales* ratAN060301C | 0.000 | 0.000 | 0.000 | 0.000 |
| Bacillaceae | 0.000 | 0.001 | 0.000 | 0.000 |
| Methylobacteriaceae | 0.000 | 0.000 | 0.000 | 0.001 |
| Comamonadaceae | 0.000 | 0.002 | 0.000 | 0.000 |
| Desulfovibrionaceae | 0.000 | 0.024 | 0.000 | 0.000 |
| Xanthomonadaceae | 0.000 | 0.057* | 0.001 | 0.000 |
| Others | 0.045 | 0.051 | 0.047 | 0.018 |

| | | | | |
|---|---|---|---|---|
| *significantly different compared to the control; significance was determined by p<0.05. | | | | |

**Table 12: Relative abundance (%) of bacterial genera in the caecum at Day 7 of the control and test mice (fed Lactobacillus gasseri APC 678, Lactobacillus rhamnosus DPC 6111 and Lactobacillus gasseri ATCC 33323).**

| Genus | Control | APC 678 | DPC 6111 | ATCC 33323 |
|---|---|---|---|---|
| uncultured Lachnospiraceae A | 29.985 | 20.933 | 14.777* | 18.000* |
| uncultured S24-7 | 24.104 | 31.182 | 40.720* | 23.214 |
| Lachnospiraceae Incertae Sedis | 9.665 | 5.321 | 4.994 | 4.906 |
| Bacteroides | 9.289 | 21.603 | 18.614 | 28.214* |
| Parabacteroides | 7.917 | 1.611 | 4.213 | 6.710 |
| Ruminococcaceae Incertae Sedis | 5.364 | 2.185* | 1.441* | 2.014* |
| uncultured Clostridiales | 2.008 | 0.639 | 0.213* | 0.582* |
| Lactobacillus | 1.835 | 1.078 | 1.259 | 1.036 |
| Akkermansia | 1.531 | 0.084 | 0.354 | 0.928 |
| Alistipes | 1.406 | 3.395* | 4.949* | 6.034* |
| uncultured Ruminococcaceae | 1.081 | 2.031* | 0.771 | 1.308 |
| Erysipelotrichaceae Incertae Sedis | 0.953 | 0.171 | 0.290 | 0.268 |
| Allobaculum | 0.691 | 0.401 | 0.449 | 0.294 |
| Peptostreptococcaceae Incertae Sedis | 0.603 | 0.078* | 0.019* | 0.050* |
| Anaerotruncus | 0.601 | 1.090 | 1.846* | 0.755 |
| Rikenellaceae RC9 gut group | 0.453 | 3.895* | 1.566* | 2.078 |
| Oscillibacter | 0.363 | 1.151* | 1.805* | 0.787 |
| Parasutterella | 0.286 | 0.085* | 0.069* | 1.235 |
| Blautia | 0.267 | 0.076 | 0.030 | 0.132 |
| Flavonifractor | 0.251 | 0.012* | 0.007* | 0.087 |
| Roseburia | 0.207 | 0.903* | 0.212 | 0.288 |
| Escherichia/Shigella | 0.177 | 0.010* | 0.017* | 0.055 |
| uncultured Erysipelotrichaceae A | 0.138 | 0.036* | 0.054* | 0.039* |
| Anaerostipes | 0.129 | 0.064 | 0.064 | 0.065 |
| Enterorhabdus | 0.128 | 0.135 | 0.159 | 0.059 |
| Bifidobacterium | 0.126 | 0.039* | 0.024* | 0.065* |
| uncultured Christensenellaceae | 0.102 | 0.375 | 0.000 | 0.008 |
| Ruminococcus | 0.082 | 0.102 | 0.001 | 0.005 |
| Coprococcus | 0.053 | 0.063 | 0.017 | 0.020 |
| Marvinbryantia | 0.038 | 0.099 | 0.047 | 0.188 |
| Enterococcus | 0.030 | 0.023 | 0.007* | 0.013 |
| Clostridiales Family XIII Incertae Sedis | 0.019 | 0.025 | 0.029 | 0.012 |
| uncultured Erysipelotrichaceae B | 0.017 | 0.009 | 0.026 | 0.011 |
| Clostridium | 0.016 | 0.000 | 0.002 | 0.001 |
| Anaeroplasma | 0.013 | 0.033 | 0.258 | 0.097 |
| Oxalobacter | 0.005 | 0.000 | 0.000 | 0.000 |
| Coprobacillus | 0.004 | 0.000 | 0.010 | 0.337 |
| uncultured Peptococcaceae | 0.004 | 0.025* | 0.006 | 0.066 |
| Acetitomaculum | 0.002 | 0.000 | 0.000 | 0.000 |
| Thalassospira | 0.002 | 0.008 | 0.000 | 0.000 |
| Staphylococcus | 0.002 | 0.287 | 0.045 | 0.000 |
| Streptococcus | 0.001 | 0.001 | 0.000 | 0.000 |
| Christensenella | 0.001 | 0.000 | 0.000 | 0.004 |
| uncultured Coriobacteriaceae | 0.001 | 0.005 | 0.007 | 0.004 |
| Idiospermum | 0.001 | 0.000 | 0.000 | 0.000 |
| uncultured Cyanobacteria 4C0d-2 | 0.001 | 0.000 | 0.000 | 0.000 |
| Acinetobacter | 0.001 | 0.005 | 0.000 | 0.000 |
| Sporosarcina | 0.000 | 0.012 | 0.000 | 0.000 |
| Barnesiella | 0.000 | 0.000 | 0.000 | 0.000 |
| Prevotella | 0.000 | 0.533 | 0.561* | 0.000 |
| uncultured Prevotellaceae | 0.000 | 0.000 | 0.000 | 0.001 |
| Rikenella | 0.000 | 0.025 | 0.000 | 0.000 |
| uncultured *Bacteroidales* ratAN060301C | 0.000 | 0.000 | 0.000 | 0.000 |
| Bacillus | 0.000 | 0.001 | 0.000 | 0.000 |
| Salinicoccus | 0.000 | 0.001 | 0.000 | 0.000 |
| Anaerovorax | 0.000 | 0.000 | 0.000 | 0.001 |
| uncultured Clostridiales Family XIII Incertae Sedis | 0.000 | 0.003 | 0.000 | 0.007 |
| uncultured Lachnospiraceae B | 0.000 | 0.002 | 0.000 | 0.000 |
| Peptococcus | 0.000 | 0.016 | 0.015 | 0.000 |
| Hydrogenoanaerobacterium | 0.000 | 0.004* | 0.000 | 0.000 |
| Subdoligranulum | 0.000 | 0.000 | 0.003 | 0.000 |
| Methylobacterium | 0.000 | 0.000 | 0.000 | 0.001 |
| Variovorax | 0.000 | 0.002 | 0.000 | 0.000 |
| Bilophila | 0.000 | 0.024 | 0.000 | 0.000 |
| Stenotrophomonas | 0.000 | 0.057* | 0.001 | 0.000 |
| Other | 0.045 | 0.051 | 0.047 | 0.018 |

| | | | | |
|---|---|---|---|---|
| *significantly different compared to the control; significance was determined by p<0.05. | | | | |

### Discussion

Because of the economic burden of the frequency of CDI in hospitals, facilities for the elderly and, more recently, community settings (Chitnis *et al.,* 2013), alternative treatments for the prevention of CDI are being investigated. The use of microbial therapy as a preventive measure is one such avenue under investigation. Surveys of the literature have shown that for paediatric use, lactobacillus strains significantly prevented antibiotic associated diarrhoea and CDI in children (Goldenberg *et al.,* 2013). However, while there was some evidence that microbial therapy was effective in preventing primary CDI in adults, there was insufficient evidence to confirm the efficacy of these strains to prevent recurrent CDI (Evans and Johnson, 2015; Goldstein *et al.,* 2015).

The concept of strain specific benefits of probiotics is not new and has also already been shown to be true of probiotics for other applications (Wall *et al.,* 2012). In this context our study pre-screened a range of *Lactobacillus* species for their ability to inhibit *C. difficile* with a view to identifying a potential strains to target CDI in humans. To that end we developed a culture medium which allowed the growth of both the *Lactobacillus* and the *C. difficile* strains in co-culture, without the drop in pH normally associated with growth of lactobacilli in other media such as MRS. We identified 4/58 *Lactobacillus* strains (2 *L. gasseri* strains APC 678 and DPC 6112, 1 *L. rhamnosus* strain DPC 6111 and 1 *L. paracasei* strain APC1483), all of human origin, which inhibited the growth of *C*. *difficile in vitro.* Among the strains screened but which did not show efficacy was *L. gasseri* ATCC 33323, which has been shown to have a number of traits encoded on its genome which are important for its survival and retention in the gastrointestinal tract (Azcarate-Peril *et al.,* 2008). *L. gasseri* APC 678 was the lead candidate from this screening and, interestingly while 10 strains *of L. gasseri* were screened *in vitro* only 2 *L. gasseri* strains, *L. gasseri* APC 678 and *L. gasseri* DPC 6112, inhibited *C. difficile* lending credence to the theory that not all strains of the same species have the same effect.

The two lead candidates from the *in vitro* work, namely *L. gasseri* APC 678 and *L. rhamnosus* DPC 6111, were selected for *in vivo* analysis. These strains had the added advantage of being capable of survival in significant numbers during simulated gastric transit, at low pH and in the presence of bile and the digestive enzymes encountered in the stomach and upper GIT. The increased survival rate in these environments in the presence of milk is a further bonus as fermented milk products such as yoghurts and cheese are often used as vehicles for oral delivery of live bacteria (Gardiner *et al.,* 1998; Hickson *et al.,* 2007).

As a first step to determine the efficacy of these strains with respect to decreasing CDI *in vivo,* the strains (*L. gasseri* APC 678, *L. rhamnosus* DPC 6111 and the aforementioned well-characterised strain *L. gasseri* ATCC 33323) were tested for their ability to reduce faecal shedding of *C. difficile* in a murine model of CDI over 7 days. The ability to reduce faecal shedding was significant, when compared to the control group fed RSM, in those animals fed *L. gasseri* APC 678 four days post infection and this reduction was maintained up to 7 days at which time the animals were euthanized. No significant effect was seen in terms of faecal shedding of C. *difficile* in those mice fed either *L. gasseri* 33323 or *L. rhamnosus* DPC 6111. Interestingly, when the level of viable *C. difficile* in the colon was assessed the numbers were significantly reduced in those mice which were fed either of the *L. gasseri* strains, which is possibly a result of competitive exclusion of *C. difficile* by the *L. gasseri* strains. The absence of evidence of bacteriocin activity *in vitro* would suggest that inhibition by bacteriocins is not the reason.

CDI is normally the result of perturbation of the gut microbiota as a result of broad-spectrum antibiotic treatment which results in a decrease in microbial diversity (Rea *et al.,* 2012b). One function of a live therapeutic in a disease state would be to increase diversity, thus reducing the ability of *C. difficile* to survive and multiply due to competition for nutrients. Compositional sequencing showed that in the control fed mice and the test groups there was a diverse microbiota despite the prior administration of antibiotics to make the animals more susceptible to infection. *L. gasseri* APC 678 increased diversity for all the indices tested, including the number of observed species compared to the other strains studied. It has been recognised that a decrease in diversity has been linked to CDI (Gu *et al.,* 2016; Rea *et al.,* 2012b). However, unlike the mice fed *L. gasseri* APC 678, where no change in the relative abundance of the main phyla was observed when compared to the control, there was a significant change in the Firmicutes and Bacteroides levels in the groups fed either *L. rhamnosus* DPC 6111 or *L. gasseri* ATCC 33323 when compared to the control with a significant increase in Bacteroidetes. In a recent paper by Schubert *et al.* (2015) it was shown that, when the gut microbiota was changed as a result of antibiotics in a murine model, populations of *Porphyromonadaceae, Lachnospiraceae, Lactobacillus* and *Alistipes* protected against *C. difficile* colonisation (Schubert et al., 2015). While our study showed that *Lachnospiraceae* were significantly reduced in the groups fed *L. rhamnosus* DPC 6111 and *L. gasseri* ATCC 33323, it was notable that *Alistipes* were significantly increased in all probiotic-fed animals relative to the control group.

In the fight against CDI it is likely that live therapeutics, either as well characterised single/multiple strains of lactobacilli and bifidobacteria as described here or the more complex less defined microbiota in FMT will play a role in addressing the reduction in microbial diversity in the GIT that results from broad spectrum antibiotic treatment leading to CDI. There are advantages of using well characterised strains with QPS status which have proven efficacy against *C. difficile in vivo* and that translate into positive changes in the gut microbiota profile. For instance, the interactions between the gut microbiome and the host are complex and FMT may therefore have unintended consequences in a patient after successful FMT due to alteration of the gut microbiota. FMT in experimental animals has shown that immunologic, behavioural and metabolic phenotypes can be transferred from donor to recipient which may not always be beneficial to the recipient in the long term. (Collins et al., 2013; Di Luccia et al., 2015; Pamer, 2014).

### Formulations

One or more of the strains of the invention may be administered to animals (including humans) in an orally ingestible form in a conventional preparation such as capsules, microcapsules, tablets, granules, powder, troches, pills, suppositories, suspensions and syrups. Suitable formulations may be prepared by methods commonly employed using conventional organic and inorganic additives. The amount of active ingredient in the medical composition may be at a level that will exercise the desired therapeutic effect.

The formulation may also include a bacterial component, a drug entity or a biological compound.

In addition a vaccine comprising one or more of the strains of the invention may be prepared using any suitable known method and may include a pharmaceutically acceptable carrier or adjuvant.

The introduction of probiotic organisms is accomplished by the ingestion of the micro-organism in a suitable carrier. It would be advantageous to provide a medium that would promote the growth of these probiotic strains in the large bowel. The addition of one or more oligosaccharides, polysaccharides, or other prebiotics enhances the growth of lactic acid bacteria in the gastrointestinal tract. Prebiotics refers to any non-viable food component that is specifically fermented in the colon by indigenous bacteria thought to be of positive value, e.g. bifidobacteria, lactobacilli. Types of prebiotics may include those that contain fructose, xylose, soya, galactose, glucose and mannose. The combined administration of a probiotic strain with one or more prebiotic compounds may enhance the growth of the administered probiotic *in vivo* resulting in a more pronounced health benefit, and is termed synbiotic.

It will be appreciated that the probiotic strains may be administered prophylactically or as a method of treatment either on its own or with other probiotic and/or prebiotic materials as described above. In addition, the bacteria may be used as part of a prophylactic or treatment regime using other active materials such as those used for treating inflammation or other disorders especially those with an immunological involvement. Such combinations may be administered in a single formulation or as separate formulations administered at the same or different times and using the same or different routes of administration.

The strains of the invention may be formulated to facilitate controlled release such as a delayed release of the strain. For example, the formulation may be adapted to release the strain at a particular location in the gastrointestinal tract such as the small intestine or in the colon. To achieve such a controlled release the strain may be formulated in a capsule which has a coating which is adapted to release the strain at a particular location. A range of coatings are available to facilitate such controlled release. One such family of coatings are those available under the Trade Mark Eudragit.

The invention is not limited to the embodiments hereinbefore described, which may be varied in detail.

### References

ABRAHAMSSON, T. R., JAKOBSSON, H. E., ANDERSSON, A. F., BJORKSTEN, B., ENGSTRAND, L. & JENMALM, M. C. 2012. Low diversity of the gut microbiota in infants with atopic eczema. J Allergy Clin Immunol, 129, 434-40, 440.e1-2.
ABRAHAMSSON, T. R., JAKOBSSON, H. E., ANDERSSON, A. F., BJORKSTEN, B., ENGSTRAND, L. & JENMALM, M. C. 2014. Low gut microbiota diversity in early infancy precedes asthma at school age. Clin Exp Allergy, 44, 842-50.
ALTERMANN, E. & KLAENHAMMER, T. R. 2003. GAMOLA: a new local solution for sequence annotation and analyzing draft and finished prokaryotic genomes. OMICS, 7, 161-9.
AZCARATE-PERIL, M. A., ALTERMANN, E., GOH, Y. J., TALLON, R., SANOZKY-DAWES, R. B., PFEILER, E. A., O'FLAHERTY, S., BUCK, B. L., DOBSON, A., DUONG, T., MILLER, M. J., BARRANGOU, R. & KLAENHAMMER, T. R. 2008. Analysis of the genome sequence of Lactobacillus gasseri ATCC 33323 reveals the molecular basis of an autochthonous intestinal organism. Appl. Environ. Microbiol., 74, 4610-4625
BACKHED, F., FRASER, C. M., RINGEL, Y., SANDERS, M. E., SARTOR, R. B., SHERMAN, P. M., VERSALOVIC, J., YOUNG, V. & FINLAY, B. B. 2012. Defining a healthy human gut microbiome: current concepts, future directions, and clinical applications. Cell Host Microbe, 12, 611-22.
BISGAARD, H., LI, N., BONNELYKKE, K., CHAWES, B. L., SKOV, T., PALUDAN-MULLER, G., STOKHOLM, J., SMITH, B. & KROGFELT, K. A. 2011. Reduced diversity of the intestinal microbiota during infancy is associated with increased risk of allergic disease at school age. J Allergy Clin Immunol, 128, 646-52.e1-5.
CAPORASO, J. G., KUCZYNSKI, J., STOMBAUGH, J., BITTINGER, K., BUSHMAN, F. D., COSTELLO, E. K., FIERER, N., PENA, A. G., GOODRICH, J. K., GORDON, J. I., HUTTLEY, G. A., KELLEY, S. T., KNIGHTS, D., KOENIG, J. E., LEY, R. E., LOZUPONE, C. A., MCDONALD, D., MUEGGE, B. D., PIRRUNG, M., REEDER, J., SEVINSKY, J. R., TURNBAUGH, P. J., WALTERS, W. A., WIDMANN, J., YATSUNENKO, T., ZANEVELD, J. & KNIGHT, R. 2010. QIIME allows analysis of high-throughput community sequencing data. Nat Methods, 7, 335-6.
CHEN, X., KATCHAR, K., GOLDSMITH, J. D., NANTHAKUMAR, N., CHEKNIS, A., GERDING, D. N. & KELLY, C. P. 2008. A mouse model of Clostridium difficile-associated disease. Gastroenterology, 135, 1984-92.
CHITNIS, A. S., HOLZBAUER, S. M., BELFLOWER, R. M., WINSTON, L. G., BAMBERG, W. M., LYONS, C., FARLEY, M. M., DUMYATI, G. K., WILSON, L. E., BELDAVS, Z. G., DUNN, J. R., GOULD, L. H., MACCANNELL, D. R., GERDING, D. N., MCDONALD, L. C. & LESSA, F. C. 2013. Epidemiology of community-associated Clostridium difficile infection, 2009 through 2011. JAMA Internal Medicine, 173, 1359-1367.
CLAESSON, M. J., CUSACK, S., O'SULLIVAN, O., GREENE-DINIZ, R., DE WEERD, H., FLANNERY, E., MARCHESI, J. R., FALUSH, D., DINAN, T., FITZGERALD, G., STANTON, C., VAN SINDEREN, D., O'CONNOR, M., HARNEDY, N., O'CONNOR, K., HENRY, C., O'MAHONY, D., FITZGERALD, A. P., SHANAHAN, F., TWOMEY, C., HILL, C., ROSS, R. P. & O'TOOLE, P. W. 2011. Composition, variability, and temporal stability of the intestinal microbiota of the elderly. Proc Natl Acad Sci U S A, 108 Suppl 1, 4586-91.
CLAESSON, M. J., JEFFERY, I. B., CONDE, S., POWER, S. E., O'CONNOR, E. M., CUSACK, S., HARRIS, H. M., COAKLEY, M., LAKSHMINARAYANAN, B., O'SULLIVAN, O., FITZGERALD, G. F., DEANE, J., O'CONNOR, M., HARNEDY, N., O'CONNOR, K., O'MAHONY, D., VAN SINDEREN, D., WALLACE, M., BRENNAN, L., STANTON, C., MARCHESI, J. R., FITZGERALD, A. P., SHANAHAN, F., HILL, C., ROSS, R. P. & O'TOOLE, P. W. 2012. Gut microbiota composition correlates with diet and health in the elderly. Nature, 488, 178-84.
CLEMENTE, J. C., PEHRSSON, E. C., BLASER, M. J., SANDHU, K., GAO, Z., WANG, B., MAGRIS, M., HIDALGO, G., CONTRERAS, M., NOYA-ALARCON, O., LANDER, O., MCDONALD, J., COX, M., WALTER, J., OH, P. L., RUIZ, J. F., RODRIGUEZ, S., SHEN, N., SONG, S. J., METCALF, J., KNIGHT, R., DANTAS, G. & DOMINGUEZ-BELLO, M. G. 2015. The microbiome of uncontacted Amerindians. Sci Adv, 1.
COLLINS, S. M., KASSAM, Z. & BERCIK, P. 2013. The adoptive transfer of behavioral phenotype via the intestinal microbiota: experimental evidence and clinical implications. Curr Opin Microbiol, 16, 240-5.
COLLINS, S. M., SURETTE, M. & BERCIK, P. 2012. The interplay between the intestinal microbiota and the brain. Nat Rev Microbiol, 10, 735-42.
COTILLARD, A., KENNEDY, S. P., KONG, L. C., PRIFTI, E., PONS, N., LE CHATELIER, E., ALMEIDA, M., QUINQUIS, B., LEVENEZ, F., GALLERON, N., GOUGIS, S., RIZKALLA, S., BATTO, J. M., RENAULT, P., DORE, J., ZUCKER, J. D., CLEMENT, K. & EHRLICH, S. D. 2013. Dietary intervention impact on gut microbial gene richness. Nature, 500, 585-8.
COTTER, P. D., STANTON, C., ROSS, R. P. & HILL, C. 2012. The impact of antibiotics on the gut microbiota as revealed by high throughput DNA sequencing. Discov Med, 13, 193-9.
CRYAN, J. F. & DINAN, T. G. 2012. Mind-altering microorganisms: the impact of the gut microbiota on brain and behaviour. Nat Rev Neurosci, 13, 701-12.
DE JONG, A., VAN HIJUM, S. A., BIJLSMA, J. J., KOK, J. & KUIPERS, O. P. 2006. BAGEL: a web-based bacteriocin genome mining tool. Nucleic Acids Res, 34, W273-9.
DEBAST, S. B., BAUER, M. P. & KUIJPER, E. J. 2014. European Society of Clinical Microbiology and Infectious Diseases: update of the treatment guidance document for Clostridium difficile infection. Clinical Microbiology and Infection, 20, 1-26.
DEPESTEL, D. D. & ARONOFF, D. M. 2013b. Epidemiology of Clostridium difficile infection. Journal of Pharmacy Practice, 26, 464-475.
DI LUCCIA, B., CRESCENZO, R., MAZZOLI, A., CIGLIANO, L., VENDITTI, P., WALSER, J. C., WIDMER, A., BACCIGALUPI, L., RICCA, E. & IOSSA, S. 2015. Rescue of Fructose-Induced Metabolic Syndrome by Antibiotics or Faecal Transplantation in a Rat Model of Obesity. PLoS One, 10, e0134893.
DINAN, T. G., STILLING, R. M., STANTON, C. & CRYAN, J. F. 2015. Collective unconscious: how gut microbes shape human behavior. Journal of Psychiatric Research, 63, 1-9. DOBSON, A., COTTER, P. D., ROSS, P. R. & HILL, C. 2012. Bacteriocin Production: a Probiotic Trait. Applied and Environmental Microbiology, 78, 1-6.
ECKBURG, P. B., BIK, E. M., BERNSTEIN, C. N., PURDOM, E., DETHLEFSEN, L., SARGENT, M., GILL, S. R., NELSON, K. E. & RELMAN, D. A. 2005. Diversity of the human intestinal microbial flora. Science, 308, 1635-8.
EDGAR, R. C. 2010. Search and clustering orders of magnitude faster than BLAST. Bioinformatics, 26, 2460-1.
EGE , M. J., MAYER , M., NORMAND , A.-C., GENUNEIT , J., COOKSON , W. O. C. M., BRAUN-FAHRLÄNDER , C., HEEDERIK , D., PIARROUX , R. & VON MUTIUS , E. 2011. Exposure to Environmental Microorganisms and Childhood Asthma. New England Journal of Medicine, 364,701-709.
EVANS, C. T. & JOHNSON, S. 2015. Prevention of Clostridium difficile infection with probiotics. Clinical Infectious Diseases, 60, S122-8.
FERNANDEZ, M., HUDSON, J. A., KORPELA, R. & DELOSREYES-GAVILAN, C. G. 2015. Impact on human health of microorganisms present in fermented dairy products: An overview. Biomed Research International, vol. 2015 Article ID 412714.
GARDINER, G. M., ROSS, P. R., COLLINS, J. K., FITZGERALD, G. & STANTON, C. 1998. Development of a probiotic cheddar cheese containing human-derived Lactobacillus paracasei strains. Applied and Environmental Microbiology, 64, 2192-2199.
GOLDENBERG, J. Z., MA, S. S., SAXTON, J. D., MARTZEN, M. R., VANDVIK, P. O., THORLUND, K., GUYATT, G. H. & JOHNSTON, B. C. 2013. Probiotics for the prevention of Clostridium difficile-associated diarrhea in adults and children. The Cochrane Database of Systematic Reviews, 5, CD006095.
GOLDSTEIN, E. J., JOHNSON, S., MAZIADE, P. J., MCFARLAND, L. V., TRICK, W., DRESSER, L., MILLETTE, M., MAZLOUM, H. & LOW, D. E. 2015. Pathway to prevention of nosocomial Clostridium difficile infection. Clin Infect Dis, 60 Suppl 2, S148-58.
GOORHUIS, A., BAKKER, D., CORVER, J., DEBAST, S. B., HARMANUS, C., NOTERMANS, D. W., BERGWERFF, A. A., DEKKER, F. W. & KUIJPER, E. J. 2008. Emergence of Clostridium difficile infection due to a new hypervirulent strain, polymerase chain reaction ribotype 078. Clin Infect Dis, 47, 1162-70.
GU, S., CHEN, Y., ZHANG, X., LU, H., LV, T., SHEN, P., LV, L., ZHENG, B., JIANG, X. & LI, L. 2016. Identification of key taxa that favor intestinal colonization of Clostridium difficile in an adult Chinese population. Microbes Infect, 18, 30-8.
HICKSON, M., D'SOUZA, A., MATHU, N., ROGERS, T. R., WANT, S., RAJKUMAR, C. & BULPITT, C. J. 2007. Use of probiotic Lactobacillus preparation to prevent diarrhoea assocated with antibiotics: randomised double blind placebo controlled trial. British Medical Journal, 335, 80-85.
IINO, T., MORI, K., TANAKA, K., SUZUKI, K. & HARAYAMA, S. 2007. Oscillibacter valericigenes gen. nov., sp. nov., a valerate-producing anaerobic bacterium isolated from the alimentary canal of a Japanese corbicula clam. Int J Syst Evol Microbiol, 57, 1840-5.
ISMAIL, I. H., OPPEDISANO, F., JOSEPH, S. J., BOYLE, R. J., LICCIARDI, P. V., ROBINS-BROWNE, R. M. & TANG, M. L. 2012. Reduced gut microbial diversity in early life is associated with later development of eczema but not atopy in high-risk infants. Pediatr Allergy Immunol, 23, 674-81.
JEFFERY, I. B., LYNCH, D. B. & O'TOOLE, P. W. 2016. Composition and temporal stability of the gut microbiota in older persons. Isme j, 10, 170-82.
KACHRIMANIDOU, M. & MALISIOVAS, N. 2011. Clostridium difficile infection: a comprehensive review. Critical Reviews in Microbiology, 37, 178-187.
KELLEY, D. R., LIU, B., DELCHER, A. L., POP, M. & SALZBERG, S. L. 2012. Gene prediction with Glimmer for metagenomic sequences augmented by classification and clustering. Nucleic Acids Res, 40, e9.
KELLY, T. N., BAZZANO, L. A., AJAMI, N. J., HE, H., ZHAO, J., PETROSINO, J. F., CORREA, A. & HE, J. 2016. Gut Microbiome Associates With Lifetime Cardiovascular Disease Risk Profile Among Bogalusa Heart Study Participants. Circ Res, 119, 956-64.
KIM, J., SMATHERS, S. A., PRASAD, P., LECKERMAN, K. H., COFFIN, S. & ZAOUTIS, T. 2008. Epidemiological features of Clostridium difficile-associated disease among inpatients at children's hospitals in the United States, 2001-2006. Pediatrics, 122, 1266-70.
KNIP, M. & SILJANDER, H. 2016. The role of the intestinal microbiota in type 1 diabetes mellitus. Nat Rev Endocrinol, 12, 154-167.
KOSTIC, ALEKSANDAR D., GEVERS, D., SILJANDER, H., VATANEN, T., HYÖTYLÄINEN, T., HÄMÄLÄINEN, A.-M., PEET, A., TILLMANN, V., POHO, P., MATTILA, I., LÄHDESMÄKI, H., FRANZOSA, ERIC A., VAARALA, O., DE GOFFAU, M., HARMSEN, H., ILONEN, J., VIRTANEN, SUVI M., CLISH, CLARY B., OREŠIČ, M., HUTTENHOWER, C., KNIP, M. & XAVIER, RAMNIK J. The Dynamics of the Human Infant Gut Microbiome in Development and in Progression toward Type 1 Diabetes. Cell Host & Microbe, 17, 260-273.
LANGILLE, M. G., ZANEVELD, J., CAPORASO, J. G., MCDONALD, D., KNIGHTS, D., REYES, J. A., CLEMENTE, J. C., BURKEPILE, D. E., VEGA THURBER, R. L., KNIGHT, R., BEIKO, R. G. & HUTTENHOWER, C. 2013. Predictive functional profiling of microbial communities using 16S rRNA marker gene sequences. Nat Biotechnol, 31, 814-21.
LE CHATELIER, E., NIELSEN, T., QIN, J., PRIFTI, E., HILDEBRAND, F., FALONY, G., ALMEIDA, M., ARUMUGAM, M., BATTO, J. M., KENNEDY, S., LEONARD, P., LI, J., BURGDORF, K., GRARUP, N., JORGENSEN, T., BRANDSLUND, I., NIELSEN, H. B., JUNCKER, A. S., BERTALAN, M., LEVENEZ, F., PONS, N., RASMUSSEN, S., SUNAGAWA, S., TAP, J., TIMS, S., ZOETENDAL, E. G., BRUNAK, S., CLEMENT, K., DORE, J., KLEEREBEZEM, M., KRISTIANSEN, K., RENAULT, P., SICHERITZ-PONTEN, T., DE VOS, W. M., ZUCKER, J. D., RAES, J., HANSEN, T., META, H. I. T. C., BORK, P., WANG, J., EHRLICH, S. D. & PEDERSEN, O. 2013. Richness of human gut microbiome correlates with metabolic markers. Nature, 500, 541-6.
LEY, R. E., TURNBAUGH, P. J., KLEIN, S. & GORDON, J. I. 2006. Microbial ecology: human gut microbes associated with obesity. Nature, 444, 1022-3.
LOZUPONE, C. A., STOMBAUGH, J. I., GORDON, J. I., JANSSON, J. K. & KNIGHT, R. 2012. Diversity, stability and resilience of the human gut microbiota. Nature, 489, 220-30.
LUCADO, J., GOULD, C. & ELIXHAUSER, A. 2006. Clostridium Difficile Infections (CDI) in Hospital Stays, 2009: Statistical Brief #124. Healthcare Cost and Utilization Project (HCUP) Statistical Briefs. Rockville (MD): Agency for Healthcare Research and Quality (US).
MAGOC, T. & SALZBERG, S. L. 2011. FLASH: fast length adjustment of short reads to improve genome assemblies. Bioinformatics, 27, 2957-63.
MARTINEZ, I., STEGEN, JAMES C., MALDONADO-GÓMEZ, MARIA X., EREN, A. M., SIBA, PETER M., GREENHILL, ANDREW R. & WALTER, J. The Gut Microbiota of Rural Papua New Guineans: Composition, Diversity Patterns, and Ecological Processes. Cell Reports, 11, 527-538.
MAYER, E. A., KNIGHT, R., MAZMANIAN, S. K., CRYAN, J. F. & TILLISCH, K. 2014. Gut microbes and the brain: paradigm shift in neuroscience. J Neurosci, 34, 15490-6.
MAYER, E. A., TILLISCH, K. & GUPTA, A. 2015. Gut/brain axis and the microbiota. J Clin Invest, 125, 926-38.
MCFARLAND, L. V. & GOH, S. 2013. Probiotics for the prevention of antibiotic associated diarrhea and Clostridium difficile infections: a meta-analysis and systematic review.
MILANI, C., TICINESI, A., GERRITSEN, J., NOUVENNE, A., LUGLI, G. A., MANCABELLI, L., TURRONI, F., DURANTI, S., MANGIFESTA, M., VIAPPIANI, A., FERRARIO, C., MAGGIO, M., LAURETANI, F., DE VOS, W., VAN SINDEREN, D., MESCHI, T. & VENTURA, M. 2016. Gut microbiota composition and Clostridium difficile infection in hospitalized elderly individuals: a metagenomic study. Sci Rep, 6, 25945.
MURPHY, E. F., COTTER, P. D., HEALY, S., MARQUES, T. M., O'SULLIVAN, O., FOUHY, F., CLARKE, S. F., O'TOOLE, P. W., QUIGLEY, E. M., STANTON, C., ROSS, P. R., O'DOHERTY, R. M. & SHANAHAN, F. 2010. Composition and energy harvesting capacity of the gut microbiota: relationship to diet, obesity and time in mouse models. Gut, 59, 1635-42.
NEBOT-VIVINUS, M., HARKAT, C., BZIOUECHE, CARTIER, H. C., PLICHON-DAINESE, R., MOUSSA, L., EUTAMENE, H., PISHVAIE, D., HOLOWACZ, S., SEYRIG, C., PICHE, T. & THEODOROU, V. 2014. Multispecies probiotic protects gut barrier function in experimental models. World J. Gastroenterol., 20, 6832-6843.
O'SHEA, E. F., COTTER, P. D., STANTON, C., ROSS, R. P. & HILL, C. 2012. Production of bioactive substances by intestinal bacteria as a basis for explaining probiotic mechanisms: bacteriocins and conjugated linoleic acid. International Journal of Food Microbiology, 152, 189-205.
O'SULLIVAN, O., COAKLEY, M., LAKSHMINARAYANAN, B., CONDE, S., CLAESSON, M. J., CUSACK, S., FITZGERALD, A. P., O'TOOLE, P. W., STANTON, C. & ROSS, R. P. 2013. Alterations in intestinal microbiota of elderly Irish subjects post-antibiotic therapy. J Antimicrob Chemother, 68, 214-21.
PAMER, E. G. 2014. Fecal microbiota transplantation: effectiveness, complexities, and lingering concerns. Mucosal Immunol, 7, 210-214.
QIN, J., LI, R., RAES, J., ARUMUGAM, M., BURGDORF, K. S., MANICHANH, C., NIELSEN, T., PONS, N., LEVENEZ, F., YAMADA, T., MENDE, D. R., LI, J., XU, J., LI, S., LI, D., CAO, J., WANG, B., LIANG, H., ZHENG, H., XIE, Y., TAP, J., LEPAGE, P., BERTALAN, M., BATTO, J.-M., HANSEN, T., LE PASLIER, D., LINNEBERG, A., NIELSEN, H. B., PELLETIER, E., RENAULT, P., SICHERITZ-PONTEN, T., TURNER, K., ZHU, H., YU, C., LI, S., JIAN, M., ZHOU, Y., LI, Y., ZHANG, X., LI, S., QIN, N., YANG, H., WANG, J., BRUNAK, S., DORE, J., GUARNER, F., KRISTIANSEN, K., PEDERSEN, O., PARKHILL, J., WEISSENBACH, J., BORK, P., EHRLICH, S. D. & WANG, J. 2010. A human gut microbial gene catalogue established by metagenomic sequencing. Nature, 464, 59-65.
QUAST, C., PRUESSE, E., YILMAZ, P., GERKEN, J., SCHWEER, T., YARZA, P., PEPLIES, J. & GLOCKNER, F. O. 2013. The SILVA ribosomal RNA gene database project: improved data processing and web-based tools. Nucleic Acids Res, 41, D590-6.
RAO, K. & SAFDAR, N. 2016. Fecal Microbiota Transplantation for the Treatment of Clostridium difficile Infection. Journal of hospital medicine, 11, 56-61.
REA, M. C., ALEMAYEHU, D., ROSS, R. P. & HILL, C. 2013. Gut solutions to a gut problem: bacteriocins, probiotics and bacteriophage for the control of Clostridium difficile infection. J. Med Microbiol. , 62, 1369-1378.
REA, M. C., O'SULLIVAN, O., SHANAHAN, F., O'TOOLE, P. W., STANTON, C., ROSS, R. P. & HILL, C. 2012b. Clostridium difficile carriage in elderly subjects and associated changes in the intestinal microbiota. J. Clinical Microbiology, 50, 867-875.
REA, M. C., SIT, C. S., CLAYTON, E., O'CONNOR, P. M., WHITTAL, R. M., ZHENG, J., VEDERAS, J. C., ROSS, R. P. & HILL, C. 2010. Thuricin CD, a novel post-translationally modified bacteriocin with a narrow spectrum of activity against Clostridium difficile. Proc Natl Acad Sci USA, 107, 9352-9357.
REMELY M, D. S., HIPPE B, ZWIELEHNER J, AUMÜLLER E, BRATH H, ET AL. 2013. Abundance and diversity of microbiota in type 2 diabetes and obesity. 253.
RIOS-COVIAN, D., RUAS-MADIEDO, P., MARGOLLES, A., GUEIMONDE, M., DE LOS REYES-GAVILAN, C. G. & SALAZAR, N. 2016. Intestinal Short Chain Fatty Acids and their Link with Diet and Human Health. Front Microbiol, 7, 185.
SCHUBERT, A. M., SINANI, H. & SCHLOSS, P. D. 2015a. Antibiotic-Induced Alterations of the Murine Gut Microbiota and Subsequent Effects on Colonization Resistance against Clostridium difficile. MBio, 6, e00974.
SHANAHAN, F. 2015. Separating the microbiome from the hyperbolome. Genome Medicine, 7, 17.
SOKOL, H., PIGNEUR, B., WATTERLOT, L., LAKHDARI, O., BERMUDEZ-HUMARAN, L. G., GRATADOUX, J. J., BLUGEON, S., BRIDONNEAU, C., FURET, J. P., CORTHIER, G., GRANGETTE, C., VASQUEZ, N., POCHART, P., TRUGNAN, G., THOMAS, G., BLOTTIERE, H. M., DORE, J., MARTEAU, P., SEKSIK, P. & LANGELLA, P. 2008. Faecalibacterium prausnitzii is an anti-inflammatory commensal bacterium identified by gut microbiota analysis of Crohn disease patients. Proc Natl Acad Sci U S A, 105, 16731-6.
SPOR, A., KOREN, O. & LEY, R. 2011. Unravelling the effects of the environment and host genotype on the gut microbiome. Nat Rev Microbiol, 9, 279-90.
STILLING, R. M., DINAN, T. G. & CRYAN, J. F. 2014. Microbial genes, brain & behaviour - epigenetic regulation of the gut-brain axis. Genes Brain Behav, 13, 69-86.
Structure, function and diversity of the healthy human microbiome. 2012. Nature, 486, 207-14.
SULTANA, R., MCBAIN, A. J. & O'NEILL, C. A. 2013. Strain-dependent augmentation of tight-junction barrier function in human primary epidermal keratinocytes by Lactobacillus and Bifidobacterium lysates. Appl. Environ. Microbiol., 76, 4887-4894.
TAP, J., MONDOT, S., LEVENEZ, F., PELLETIER, E., CARON, C., FURET, J. P., UGARTE, E., MUNOZ-TAMAYO, R., PASLIER, D. L., NALIN, R., DORE, J. & LECLERC, M. 2009. Towards the human intestinal microbiota phylogenetic core. Environ Microbiol, 11, 2574-84.
THOMAS, L. V., OCKHUIZEN, T. & SUZUKI, K. 2014. Exploring the influence of the gut microbiota and probiotics on health: a symposium report. Br JNutr, 112 Suppl 1, S1-18.
TURNBAUGH, P. J., BACKHED, F., FULTON, L. & GORDON, J. I. 2008. Diet-induced obesity is linked to marked but reversible alterations in the mouse distal gut microbiome. Cell Host Microbe, 3, 213-23.
TURNBAUGH, P. J., HAMADY, M., YATSUNENKO, T., CANTAREL, B. L., DUNCAN, A., LEY, R. E., SOGIN, M. L., JONES, W. J., ROE, B. A., AFFOURTIT, J. P., EGHOLM, M., HENRISSAT, B., HEATH, A. C., KNIGHT, R. & GORDON, J. I. 2009. A core gut microbiome in obese and lean twins. Nature, 457, 480-4.
VAN NIMWEGEN, F. A., PENDERS, J., STOBBERINGH, E. E., POSTMA, D. S., KOPPELMAN, G. H., KERKHOF, M., REIJMERINK, N. E., DOMPELING, E., VAN DEN BRANDT, P. A., FERREIRA, I., MOMMERS, M. & THIJS, C. 2011. Mode and place of delivery, gastrointestinal microbiota, and their influence on asthma and atopy. J Allergy Clin Immunol, 128, 948-55.e1-3.
VARDAKAS, K. Z., KONSTANTELIAS, A. A., LOIZIDIS, G., RAFAILIDIS, P. I. & FALAGAS, M. E. 2012. Risk factors for development of Clostridium difficile infection due to BI/NAP1/027 strain: a meta-analysis. Int J Infect Dis, 16, e768-73.
VAZQUEZ-BAEZA, Y., PIRRUNG, M., GONZALEZ, A. & KNIGHT, R. 2013. EMPeror: a tool for visualizing high-throughput microbial community data. Gigascience, 2, 16.
VRIEZE, A., VAN NOOD, E., HOLLEMAN, F., SALOJARVI, J., KOOTTE, R. S., BARTELSMAN, J. F., DALLINGA-THIE, G. M., ACKERMANS, M. T., SERLIE, M. J., OOZEER, R., DERRIEN, M., DRUESNE, A., VAN HYLCKAMA VLIEG, J. E., BLOKS, V. W., GROEN, A. K., HEILIG, H. G., ZOETENDAL, E. G., STROES, E. S., DE VOS, W. M., HOEKSTRA, J. B. & NIEUWDORP, M. 2012. Transfer of intestinal microbiota from lean donors increases insulin sensitivity in individuals with metabolic syndrome. Gastroenterology, 143, 913-6.e7.
WALKER, A. W., INCE, J., DUNCAN, S. H., WEBSTER, L. M., HOLTROP, G., ZE, X., BROWN, D., STARES, M. D., SCOTT, P., BERGERAT, A., LOUIS, P., MCINTOSH, F., JOHNSTONE, A. M., LOBLEY, G. E., PARKHILL, J. & FLINT, H. J. 2011. Dominant and diet-responsive groups of bacteria within the human colonic microbiota. Isme j, 5, 220-30.
WALL, R., MARQUES, T. M., O'SULLIVAN, O., ROSS, R. P., SHANAHAN, F., QUIGLEY, E. M., DINAN, T. G., KIELY, B., FITZGERALD, G. F., COTTER, P. D., FOUHY, F. & STANTON, C. 2012. Contrasting effects of Bifidobacterium breve NCIMB 702258 and Bifidobacterium breve DPC 6330 on the composition of murine brain fatty acids and gut microbiota. Am J Clin Nutr, 95, 1278-87.
WIEGAND, P. N., NATHWANI, D., WILCOX, M. H., STEPHENS, J., SHELBAYA, A. & HAIDER, S. 2012. Clinical and economic burden of Clostridium difficile infection in Europe: a systematic review of healthcare-facility-acquired infection. Journal of Hospital Infection, 81, 1-14.
WILCOX, M. H., MOONEY, L., BENDALL, R., SETTLE, C. D. & FAWLEY, W. N. 2008. A case-control study of community-associated Clostridium difficile infection. J Antimicrob Chemother, 62, 388-96.
WORLD HEALTH ORGANIZATION. 2016. *Non communicable diseases.* [ONLINE] Available at: http://www.who.int/mediacentre/factsheets/fs355/en/. [Accessed 10 November 2016].
WORLD HEALTH ORGANIZATION. 2016. The top 10 causes of death. [ONLINE] Available at: http://www.who.int/mediacentre/factsheets/fs310/en/. [Accessed 10 November 2016].
YUTIN, N. & GALPERIN, M. Y. 2013. A genomic update on clostridial phylogeny: Gram-negative spore formers and other misplaced clostridia. Environ Microbiol, 15, 2631-41.
ZHOU, Y., LIANG, Y., LYNCH, K. H., DENNIS, J. J. & WISHART, D. S. 2011. PHAST: a fast phage search tool. Nucleic Acids Res, 39, W347-52.
ZIAKAS, P. D., ZACHARIOUDAKIS, I. M., ZERVOU, F. N., GRIGORAS, C., PLIAKOS, E. E. & MYLONAKIS, E. 2015. Asymptomatic carriers of toxigenic C. difficile in long-term care facilities: a meta-analysis of prevalence and risk factors. PLoS One, 10, e0117195.

## Claims

1. A strain selected from one or more of:
*Lactobacillus gasseri* strain APC678 having NCIMB accession number 42658;
*Lactobacillus rhamnosus* DPC6111 having NCIMB accession number 42661;
*Lactobacillus gasseri* strain DPC6112 having NCIMB accession number 42659; and
*Lactobacillus paracasei* strain APC1483 having NCIMB accession number 42660 for use in the prophylaxis and/or treatment of a *Clostridium difficile* colonisation or infection.

2. An isolated strain of *Lactobacillus gasseri* APC678 (NCIMB 42658).

3. An isolated strain of *Lactobacillus rhamnosus* DPC6111 (NCIMB 42661).

4. An isolated strain of *Lactobacillus gasseri* DPC6112 (NCIMB 42659).

5. An isolated strain of *Lactobacillus paracasei* APC1483 (NCIMB 42660).

6. A mutant or variant of a strain as claimed in any of claims 2 to 5.

7. A formulation comprising one or more strains of claims 2 to 6.

8. A formulation as claimed in claim 7 which comprises an ingestible carrier such as a pharmaceutically acceptable carrier or a food product which may be selected from the group comprising acidified milk, yoghurt, frozen yoghurt, milk powder, milk concentrate, cheese spread, dressing and beverage.

9. A strain as claimed in any of claims 2 to 6 or a formulation as claimed in claim 7 or 8 for use in the prophylaxis and/or treatment of a *Clostridium difficile* colonisation or infection.

10. A strain as claimed in any of claims 2 to 6 or a formulation as claimed in claim 7 or 8 for use in the prophylaxis or treatment of low bacterial biodiversity of the gastrointestinal tract.

11. A strain as claimed in any of claims 2 to 6 or a formulation as claimed in claim 7 or 8 for use in association with antibiotic treatment.

12. A method for screening a bacterial strain such as a *Lactobacillus* for activity against C. *difficile* comprising:
co-culturing a strain of interest with C. *difficile* in a co-culture medium comprising sugars (such as glucose) in a concentration of from 0.01 g/l to 2.0 g/l, the co-culture medium having a pH of from 6.7 to 6.9.

13. A method as claimed in claim 12 wherein :-
the co-culture medium has a pH of 6.8;
the co-culture medium comprises sugars (such as glucose) in a concentration of from 0.01 g/l to 1.0 g/l; and/or
the co-culture medium comprises sugars (such as glucose) in a concentration of from 0.05 g/l to 0.5 g/l; and/or
the co-culture medium comprises sugars (such as glucose) in a concentration of from 0.09 g/l to 0.11 g/l.

14. A co-culture medium for use in screening a bacterial strain for activity against C. *difficile* wherein the medium comprises sugars (such as glucose) in a concentration of from 0.01 g/l to 2.0 g/l and wherein the co-culture medium has a pH of from 6.7 to 6.9.

15. A co-culture medium as claimed in claim 14 wherein:-
the co-culture medium has a pH of 6.8;
the co-culture medium comprises sugars (such as glucose) in a concentration of from 0.01 g/l to 1.0 g/l; and/or
the co-culture medium comprises sugars (such as glucose) in a concentration of from 0.05 g/l to 0.5 g/l; and/or
the co-culture medium comprises sugars (such as glucose) in a concentration of from 0.09 g/l to 0.11 g/l.


<110>  UNIVERSITY COLLEGE CORK - NATIONAL UNIVERSITY OF IRELAND,
       CORK
       TEASASC, THE AGRICULTURAL AND FOOD DEVELOPMENT AUTHORITY

<120>  Human lactobacilli strains

<130>  UCC009/P

<160>  5

<170>  PatentIn version 3.5

<210>  1
<211>  27
<212>  DNA
<213>  Artificial Sequence

<220>
<223>  synthesised sequence

<400>  1
ctgatctcga gggcggtgtg tacaagg                                          27


<210>  2
<211>  30
<212>  DNA
<213>  Artificial Sequence

<220>
<223>  synthesised sequence

<400>  2
ctgatgaatt cgagacacgg tccagactcc                                       30


<210>  3
<211>  1026
<212>  DNA
<213>  Lactobacillus gasseri

<400>  3
cgatactagc gattccgctt cgtgtaggcg agttgcagcc tacagtccga actgagaacg      60

gctttcagag atccgcttgc cttcgcaggt tcgcttctcg ttgtaccgtc cattgtagca     120

cgtgtgtagc ccaggtcata aggggcatga tgacttgacg tcatccccac cttcctccgg     180

tttgtcaccg gcagtctcat tagagtgccc aacttaatga tggcaactaa tgacaagggt     240

tgcgctcgtt gcgggactta acccaacatc tcacgacacg agctgacgac agccatgcac     300

cacctgtctc agcgtccccg aagggaacac ctaatctctt aggtttgcac tggatgtcaa     360

gacctggtaa ggttcttcgc gttgcttcga attaaaccac atgctccacc gcttgtgcgg     420

gcccccgtca attcctttga gtttcaacct tgcggtcgta ctccccaggc ggagtgctta     480

atgcgttagc tgcagcactg agaggcggaa acctcccaac acttagcact catcgtttac     540

ggcatggact accagggtat ctaatcctgt tcgctaccca tgctttcgag cctcagcgtc     600

agttgcagac cagagagccg ccttcgccac tggtgttctt ccatatatct acgcattcca 660

ccgctacaca tggagttcca ctctcctctt ctgcactcaa gttcaacagt ttctgatgca 720

attctccggt tgagccgaag gctttcacat cagacttatt gaaccgcctg cactcgcttt 780

acgcccaata aatccggaca acgcttgcca cctacgtatt accgcggctg ctggcacgta 840

gttagccgtg actttctaag taattaccgt caaataaagg ccagttacta cctctatctt 900

tcttcactac caacagagct ttacgagccg aaacccttct tcactcacgc ggcgttgctc 960

catcagactt gcgtccattg tggaagattc cctactgctg cctcccgtag gagtctggac 1020

cgtgtc 1026

<210> 4
<211> 50
<212> DNA
<213> Artificial Sequence

<220>
<223> synthesised sequence

<220>
<221> misc_feature
<222> (42)..(42)
<223> n is a, c, g, or t

<400> 4
tcgtcggcag cgtcagatgt gtataagaga cagcctacgg gnggcwgcag 50

<210> 5
<211> 55
<212> DNA
<213> Artificial Sequence

<220>
<223> synthesised sequence

<400> 5
gtctcgtggg ctcggagatg tgtataagag acaggactac hvgggtatct aatcc 55